# EUROPEAN PATENT SPECIFICATION

(11) **EP 4 116 408 B1**
(45) Date of publication and mention of the grant of the patent: **23.10.2024**
(21) Application number: 22180001.4
(22) Date of filing: 20.06.2022
(51) Int. Cl.: C12N 1/16, C12N 15/63, C12N 15/81, C12P 21/02

(54) **METHOD FOR METHANOL FREE CULTURING OF METHYLOTROPHIC YEAST FOR THE BIOSYNTHESIS OF ADDED VALUE PRODUCTS**
VERFAHREN ZUR METHANOLFREIEN KULTIVIERUNG VON METHYLOTROPHER HEFE FÜR DIE BIOSYNTHESE VON PRODUKTEN MIT MEHRWERT
PROCÉDÉ DE CULTURE SANS MÉTHANOL DE LEVURE MÉTHYLOTROPHES POUR LA BIOSYNTHÈSE DE PRODUITS À VALEUR AJOUTÉE

(30) Priority: 24.06.2021 US 202163214376 P; 17.09.2021 US 202117478241
(43) Date of publication of application: 11.01.2023
(73) Proprietor: BioBoost Synbio Consulting Inc., Burnaby, Bristish Columbia V5H 4E9 (CA)
(72) Inventor: Tyurin, Oleg, Burnaby, V5H 4E9 (CA); Sun, Mingyang, Burnaby, V5H 4E9 (CA)
(74) Representative: Franke, Dirk

(56) References cited:
- WO-A1-2017/109082
- VOGL THOMAS ET AL: "Orthologous promoters from related methylotrophic yeasts surpass expression of endogenous promoters of Pichia pastoris", AMB EXPRESS, vol. 10, no. 1, 25 February 2020 (2020-02-25), XP093003617, Retrieved from the Internet <URL:http://link.springer.com/article/10.1186/s13568-020-00972-1/fulltext.html> DOI: 10.1186/s13568-020-00972-1
- TYURIN O. V. ET AL: "Deletion of the FLD gene in methylotrophic yeasts Komagataella phaffii and Komagataella kurtzmanii results in enhanced induction of the AOX1 promoter in response to either methanol or formate", MICROBIOLOGY, vol. 84, no. 3, 1 May 2015 (2015-05-01), US, pages 408 - 411, XP093002705, ISSN: 0026-2617, Retrieved from the Internet <URL:http://link.springer.com/article/10.1134/S0026261715030212/fulltext.html> DOI: 10.1134/S0026261715030212
- SINGH ANAMIKA ET AL: "The Mut+ strain of Komagataella phaffii (Pichia pastoris) expresses PAOX1 5 and 10 times faster than MutS and Mut- strains: evidence that formaldehyde or/and formate are true inducers of PAOX1.", APPLIED MICROBIOLOGY AND BIOTECHNOLOGY, SPRINGER BERLIN HEIDELBERG, BERLIN/HEIDELBERG, vol. 104, no. 18, 6 August 2020 (2020-08-06), pages 7801 - 7814, XP037226411, ISSN: 0175-7598, [retrieved on 20200806], DOI: 10.1007/S00253-020-10793-8

## Description

### PRIOR APPLICATION INFORMATION

The instant application claims the benefit of US Patent Application Serial Number 17/478,241, filed September 17, 2021, which claims the benefit of US Provisional Patent Application 63/214,376, filed June 24, 2021 and entitled "Method for methanol free culturing of methylotrophic yeast for the biosynthesis of added value products".

### FIELD OF THE INVENTION

The present invention relates to the field of culturing/fermentation of methylotrophic yeast (e.g. *Pichia pastoris* or *Komagataella phaffii*) to produce value added products like recombinant proteins or small molecule compounds.

### BACKGROUND

Methylotrophic yeast such as *Pichia pastoris,* also known as *Komagataella phaffii,* is widely used as a host organism for recombinant protein production. The ability of this yeast to use the cheap substrate methanol as sole carbon source, its high cell density fermentation capability, its secretory machinery and powerful and tightly regulated promoters have resulted in its extensive application in biotechnology. Albeit there are strong constitutive promoters like GAP (Glyceraldehyde-3-P dehydrogenase) promoter, TEF1 promoter (Translation elongation factor ), inducible promoters like AOX1 promoter (Alcohol oxidase) have advantages for production purposes as they allow biomass growth without product formation. Thus, cells are not stressed by the accumulation of recombinant products during growth, enabling better process control. AOX1 gene in *P. pastoris* cells is dramatically upregulated in response to methanol, whereas it stays tightly repressed when glucose or glycerol or any other fermentable carbon source is in the media. Therefore, amongst all inducible promoters, AOX1 promoter is most abundantly used. A typical bioprocess driven by any inducible promoter comprises biomass growth on glucose or glycerol (batch) phase followed by induction of expression followed by recombinant protein(s)/compound(s) production upon switching to methanol (fed-batch and induction phase).

In response to different carbon sources, all inducible promoters, including AOX1, have three regulated states of gene expression: catabolite repression (or just repression), derepression, and activation (induction). For AOX1 promoters, these states are well described, unlike for the other inducible promoters described herein. When glycerol or glucose or ethanol or any other fermentable carbon source is in abundance in the culture media, AOX1 promoter is completely repressed. When those carbon sources are depleted, the AOX1 promoter is derepressed, which means it is activated at roughly 2-5% of its methanol induction level. AOX1 promoter can also be de-repressed when culture grows on so-called non-repressible carbon sources (e.g. sorbitol, mannitol, alanine or trehalose). When the culture starts to consume methanol as a carbon source, with or without non-repressible carbon sources, AOX1 promoter, and the other listed promoters are fully activated (i.e. induced), Although methanol is widely used as an inducer for the AOX1 driven induction system, many shortcomings of using methanol in the fermentation process (e.g. it's flammability and toxicity) greatly limits the feasibility of this system at large industrial scales. Recently it was shown that the salts of formic acid (formates) can induce AOX1 promoter almost as well as methanol. However, it still remains unknown whether other native promoters discussed herein can be induced by formic acid or formates. Also, it has not been determined if formates or formic acid can be used as an inducer in combination with non-repressible carbon sources.

The international patent publication WO 2017/109082 A1 discloses a yeast cell of the *Komagataella* genus comprising an orthologous promoter of a methylotrophic yeast cell or a variant thereof inducible by depression.

The scientific publication T. Vogl et al., AMB Express 2020, 10, 1 discloses orthologous promoters from related methylotrophic yeasts surpassing expression of endogenous promoters of *Pichia pastoris.*

The scientific publication O. V. Tyurin et al., Microbiology 2015 84, 3, 408-411, discloses the deletion of the FLD gene in methylotrophic yeasts *Komagataella phaffii* and *Komagataella kurtzmanii* resulting in enhanced induction of the AOX1 promoter in response to either methanol or formate.

The scientific publication A. Singh et al., Applied Microbiol. Biotechnol. 2020, 104, 18, 7801-7814. discloses the Mut⁺ strain of *Komagataella phaffii* expressing O_{AOX1} 5 ad 10 times faster than Mut^{S} and Mut⁻ strains.

### SUMMARY OF THE INVENTION

The safety aspect of the fermentation / culturing process is supposed to be the subject of particular attention. In this respect, the storage of large volumes of hazardous and flammable methanol at industrial facilities is highly undesirable. Apart from the fact that it makes the fermentation process dangerous and environmentally un-friendly, the expenses for extra safety measures can add an extra 15% to total production cost. Furthermore, methanol metabolism leads to an increase in heat evolution, which is not technologically favorable because the culturing consumes a lot of energy to chill the bioreactors. Another downside of methanol metabolism is a high oxygen consumption by the culture, which is considered as a hazard because it requires a production facility with a high oxygen capacity. Thus, to make the bioprocess safer and cheaper, it is highly advantageous to exclude methanol from the culturing process, while maintaining or even surpassing the high level of expression of the conventional AOX1 or other MUT pathway inducible genes. There are some currently used approaches based on genetic modifications of yeast, but none allows for complete avoidance of methanol use with already designed strains.

Described herein is a culturing method that combines the use of the previously described alternative inducing agent, salts of formic acid (formates) or formic acid together with any suitable non-repressing feeding substrate, such as sorbitol, mannitol, trehalose or alanine. Also described is the use of salts of formic acid (formates) or formic acid as an alternative to methanol inducer with or without non-repressing carbon sources for the following promoters: the NAD⁺-dependent formate dehydrogenase (FDH) promoter (one example of which is provided as SEQ ID NO: 1), the alcohol oxidase 2 (AOX2) promoter (one example of which is provided as SEQ ID NO:2), peroxin Pex14p (PEX14) promoter (one example of which is provided as SEQ ID NO:3), the dihydroxyacetone kinase (DAK) promoter (one example of which is provided as SEQ NO:ID 5), the dihydroxyacetone synthase 1,2 (DAS1,2) promoter (example of which are provided as SEQ ID NO: 10 SEQ ID NO: 11 respectively), the formyl-glutathione dehydrogenase (FGH) promoter (one example of which is provided as SEQ ID NO:4), the formaldehyde dehydrogenase 1 (FLD1) promoter (one example of which is provided as SEQ ID NO: 12), the Fructose 1,6-bisphosphate aldolase (FBA) promoter (one example of which is provided as SEQ ID NO:6), the Peroxisomal membrane signal receptor PTS1 (PEX5) promoter (one example of which is provided as SEQ ID NO:7), the alcohol dehydrogenase 2 (ADH2) promoter (one example of which is provided as SEQ ID NO:8), and a catalase (CAT) promoter (one example of which is provided as SEQ ID NO:9 ). As will be apparent to those of skill in the art, the promoter sequences provided in SEQ ID NOs: 1-12 are intended for illustrative purposes only and suitable variants of these may be used within the invention, depending on the host cell used.

As will be appreciated by one of skill in the art, finding a new compound that is capable of induction of certain promoter(s) is not obvious from the perspective that it needs to be demonstrated that the conditions work. Accordingly, the fact that methanol works as an inducer with some non-repressive substrates does not mean at all that formates (or formic acid) should work the same way. This is especially true as the mechanism of activation of the promoters is not yet clear. Consequently, no one can say with absolute certainty that a metabolite can be an inducer under certain growth conditions. While some regulatory transcription activation factors were elucidated for the intensively used AOX1 promoter, nothing is shown for all the other promoters of the MUT (Methanol UTilization) pathway examples of which are listed above (SEQ ID NOs:1-9).

According to an aspect of the invention, there is provided a method for producing a transgenic cell product as specified in claim 1.

### BRIEF DESCRIPTION OF THE DRAWINGS

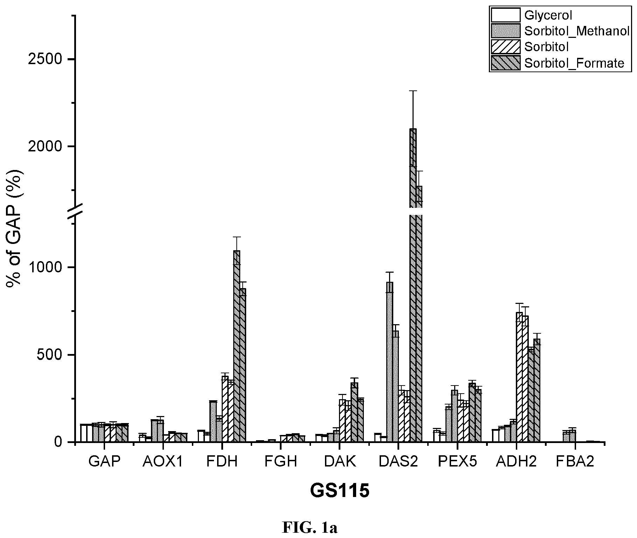

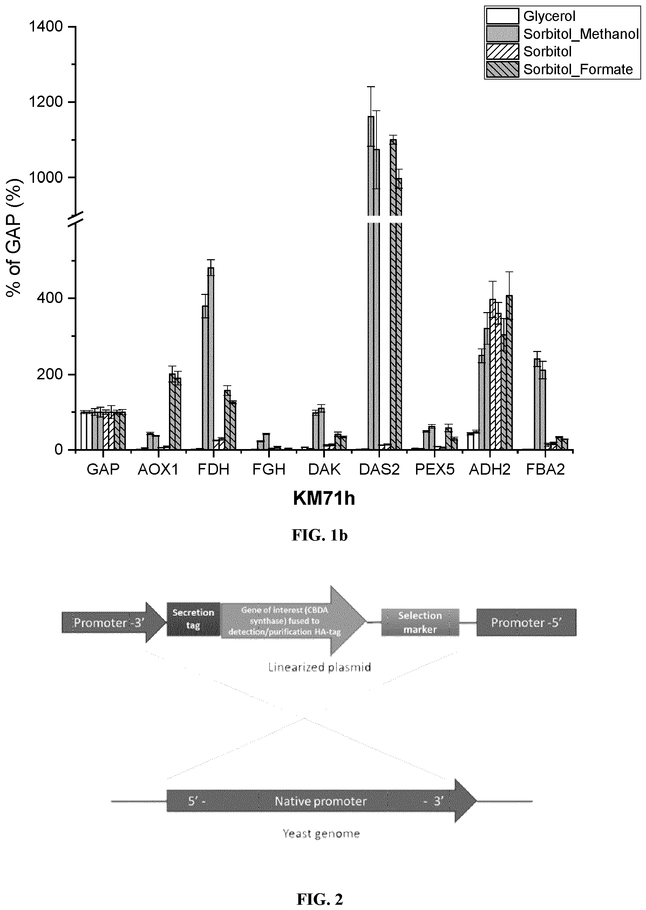

FIG. 1 Chart for promoters induction levels in response to glycerol, sorbitol, methanol and potassium formate, a) for GS115 strain; b) for KM71h strain
FIG. 2 Schematic illustration integration of an expression cassette in yeast strain GS115 genome.

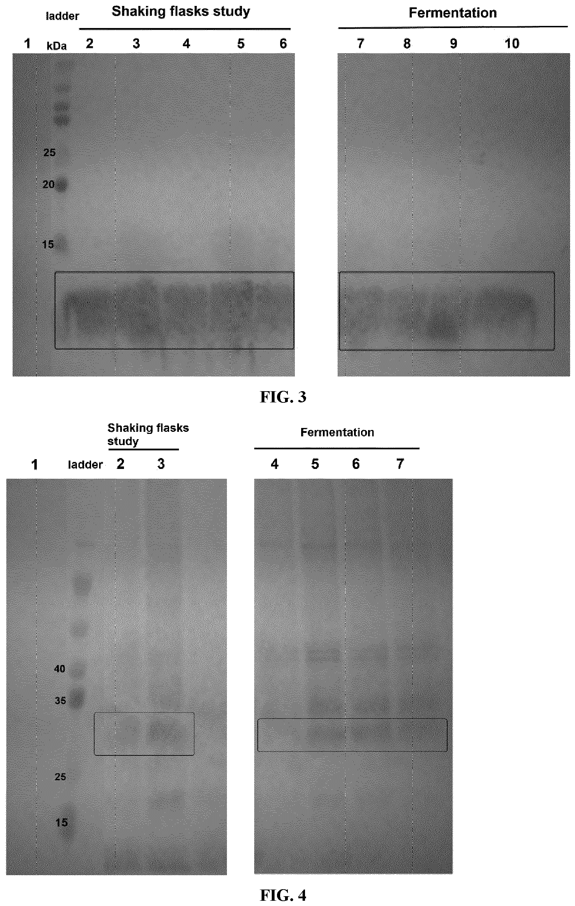

FIG. 3 Western blot of the samples from shaking flasks experiments and fermentation of GS115/pL_hEGF; 1. GS115 (negative control); protein ladder (catalog no. BZ0011G, BioBasic, Canada); 2. GS115/pL_hEGF clone#1 methanol induced culture; 3-6. GS115/pL_hEGF clones #1-4 potassium formate induction; 7. GS115/pL_hEGF fermentation samples with methanol induction /48 hours of culturing; 8. GS115/pL_hEGF fermentation samples with methanol induction /72 hours of culturing; 9. GS115/pL_hEGF fermentation samples with potassium formate induction /48 hours of culturing; 10. GS115/pL_hEGF fermentation samples with potassium formate induction /72 hours of culturing
FIG. 4 Western blot of the samples from shaking flasks experiments and fermentation of GS115/pL_hSOD3; 1. GS115 (negative control); protein ladder (catalog no. BZ0011G, BioBasic, Canada); 2. GS115/pL_hSOD3 methanol induced culture; 3. GS115/pL_hSOD3 potassium formate induction; 4. GS115/pL_hSOD3 fermentation samples with methanol induction /48 hours of culturing; 5. GS115/pL_hSOD3 fermentation samples with methanol induction /72 hours of culturing; 6. GS115/pL_hSOD3 fermentation samples with potassium formate induction /48 hours of culturing; 7. GS115/pL_hSOD3 fermentation samples with potassium formate induction /72 hours of culturing

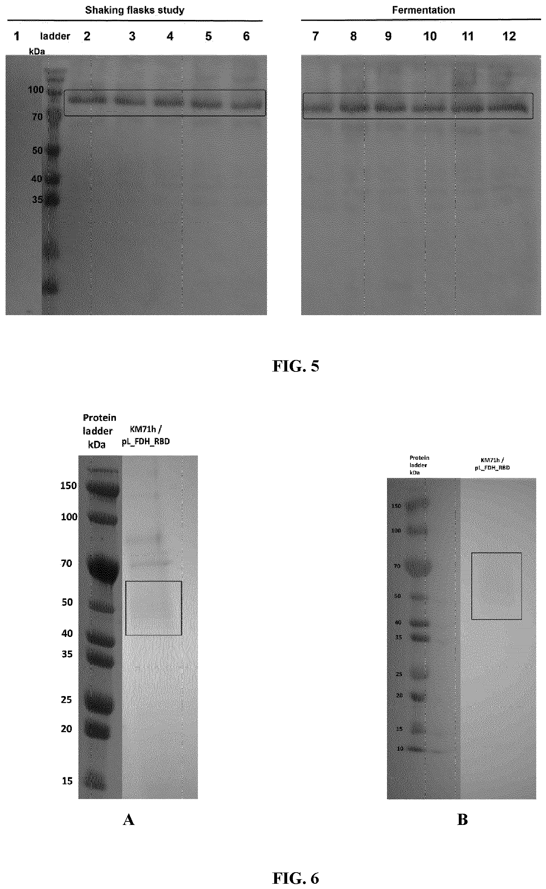

FIG. 5 Western blot of the samples from shaking flasks experiments and fermentation of GS115/pL_hLF; 1. GS115 (negative control); protein ladder (catalog no. BZ0011G, BioBasic, Canada); 2. GS115/pL_hLF clone#1 methanol induced culture; 3-6. GS115/pL_hLF clones #1-4 potassium formate induction; 7. GS115/pL_hLF fermentation samples with methanol induction /24 hours of culturing; 8. GS115/pL_hLF fermentation samples with methanol induction /48 hours of culturing; 9. GS115/pL_hLF fermentation samples with methanol induction /72 hours of culturing; 10. GS115/pL_hLF fermentation samples with potassium formate induction /24 hours of culturing; 11. GS115/pL_hLF fermentation samples with potassium formate induction /48 hours of culturing; 12. GS 115/pL_hLF fermentation samples with potassium formate induction /72 hours of culturing
FIG. 6 A. SDS-PAGE of the sample from shaking flasks experiment. Protein ladder (catalog no. BZ0011G, BioBasic, Canada); GS115/pL_RBD potassium formate induction; B. Western blot of the sample from shaking flasks experiment. Protein ladder (catalog no. BZ0011G, BioBasic, Canada); GS115/pL_RBD potassium formate induction;

### DETAILED DESCRIPTION

Unless otherwise defined, scientific and technical terms used herein have the meanings that are commonly understood by those of ordinary skill in the art. In the event of any latent ambiguity, definitions provided herein take precedent over any dictionary or extrinsic definition. Unless otherwise required by context, singular terms shall include pluralities and plural terms shall include the singular. The use of "or" means "and/or" unless stated otherwise. The use of the term "including," as well as other forms, such as "includes" and "included," is not limiting.

Generally, nomenclature used in connection with cell culture, molecular biology, immunology, microbiology, genetics, and protein and nucleic acid chemistry described herein is well-known and commonly used in the art. The methods and techniques provided herein are generally performed according to conventional methods well known in the art and as described in various general and more specific references that are cited and discussed throughout the present specification unless otherwise indicated. The nomenclatures used in connection with, and the laboratory procedures and techniques of molecular biology described herein are those well-known and commonly used in the art.

That the disclosure may be more readily understood, select terms are defined below.

The articles "a" and "an" are used herein to refer to one or to more than one (i.e., to at least one) of the grammatical object of the article. By way of example, "an element" means one element or more than one element.

All numerical designations, e.g., pH, temperature, time, concentration, amounts, and molecular weight, including ranges, are approximations which are varied (+) or (-) by 10%, 1%, or 0.1%, as appropriate. It is to be understood, although not always explicitly stated, that all numerical designations may be preceded by the term "about." It is also to be understood, although not always explicitly stated, that the reagents described herein are merely exemplary and that equivalents of such are known in the art.

The term "comprising" or "comprises" is intended to mean that the compositions and methods include the recited elements, but do not exclude others. "Consisting essentially of," when used to define compositions and methods, shall mean excluding other elements of any essential significance to the combination. For example, a composition consisting essentially of the elements as defined herein would not exclude other elements that do not materially affect the basic and novel characteristic(s) of the claimed invention. "Consisting of" shall mean excluding more than a trace amount of other ingredients and substantial method steps recited. Embodiments defined by each of these transition terms are within the scope of this invention.

The use of the term "or" in the claims is used to mean "and/or" unless explicitly indicated to refer to alternatives only, or the alternatives are mutually exclusive, although the disclosure supports a definition that refers to only alternatives and "and/or."

As used herein, the term "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10% of the particular term.

The term "polynucleotide" refers to a double-stranded or single-stranded DNA, as well as complementary nucleic acid sequences. Polynucleotide includes a sequence of nucleoside or nucleotide monomers consisting of naturally occurring bases, sugars, and intersugar (backbone) linkages. The term also includes modified or substituted sequences comprising non-naturally occurring monomers or portions thereof. The nucleic acid sequences of the present disclosure may be deoxyribonucleic acid sequences (DNA) or ribonucleic acid sequences (RNA) and may include naturally occurring bases including adenine, guanine, cytosine, thymidine, and uracil. The sequences may also contain modified bases.

The term "protein" or "polypeptide" refers to a sequence of amino acid residues encoded by a nucleic acid molecule. Within the context of the present application, a polypeptide of the disclosure may in one embodiment include various structural forms of the primary protein. For example, a polypeptide of the disclosure may be in the form of acidic or basic salts or in neutral form. In addition, individual amino acid residues may be modified by oxidation or reduction. The proteins and polypeptides of the present disclosure may also include truncations, analogs, and homologs of the proteins and polypeptides as described herein having substantially the same function as the proteins or polypeptides of the present disclosure.

As used herein "construct" or "plasmid" refer to an artificially created nucleic acid, comprising a delivery vector and a gene(s) of interest, for example a vector comprising a polynucleotide described herein. The polynucleotide of interest can be cloned into a plasmid of interest to produce a construct. In an embodiment, the vector is an expression vector. Possible expression vectors include but are not limited to cosmids or plasmids, so long as the vector is compatible with the host cell used. The expression vectors are suitable for transformation of a host cell, which means that the expression vectors contain a polynucleotide such as those exemplified in the application and regulatory sequences selected on the basis of the host cells to confer the expression of a gene of interest.

Operatively linked (or operably linked) is intended to mean that the gene of interest is linked to regulatory sequences in a manner which allows expression of this gene of interest. In some embodiments, the isolated and/or purified nucleic acid molecules, polynucleotides or vectors, constructs, or in vitro expression systems comprising these isolated and/or purified nucleic acid molecules, may be used to create transgenic or recombinant organisms or recombinant cells (e.g. optionally cells of recombinant organisms) that produce polypeptides or any small molecule compound. A nucleotide sequence is associated in a manner of receiving, for example, a promoter is operably linked to a coding sequence of a recombinant gene when it can affect the expression of the coding sequence.

Described herein is an expression system for the production of for example recombinant proteins or small molecules, as discussed herein. In some embodiments, the expression system comprises a recombinant vector or a part thereof as disclosed herein. In some embodiments, the expression system comprises a suitable host cell, for example, a microbial cell, a yeast cell, a plant cell, or an animal cell. In another embodiment, the host expression system comprises a yeast cell. In one embodiment, the yeast cell comprises one or more of *Pichia pastoris, Komagataella kurtzmanii, Komagataella phaffii, Pichia angusta, Pichia guillermordii, Pichia methanolica, Pichia inositovera, Hansenula polymorpha, Candida boidinii,* and *Yarrowia lipolytica.*

As used herein "batch phase" refers to the first phase of culturing / fermentation upon inoculation where the culture grows to reach the needed optical density (OD₆₀₀) before induction phase. As discussed herein and as will be apparent to one of skill in the art, the specific OD₆₀₀ will depend on several factors, including but by no means limited to the transgenic cell product of interest, for example, a foreign peptide or small molecule, being produced; and the host cell being used.

As used herein "induction phase" or "fed-batch phase" or "continuous phase" refer to the second phase of culturing following the batch phase, where the culture is induced, which can be considered as switching on the expression of gene(s) of interest by adding a compound called an inducer.

According to an aspect of the invention, there is provided a method for producing a transgenic cell product as specified in claim 1.

The inducible promoter may be selected from the group consisting of: NAD+-dependent formate dehydrogenase (FDH) promoter (one example of which is provided as SEQ ID NO: 1); alcohol oxidase 2 (AOX2) promoter (one example of which is provided as SEQ ID NO:2); dihydroxyacetone kinase (DAK) promoter (one example of which is provided as SEQ ID NO: 5); dihydroxyacetone synthase 1,2 (DAS1,2) promoter (examples of which are provided as SEQ ID NO:10 and SEQ ID NO:11 respectively); formaldehyde dehydrogenase 1 (FLD1) promoter (one example of which is provided as SEQ ID NO: 12); Fructose 1,6-bisphosphate aldolase (FBA) promoter (one example of which is provided as SEQ ID NO:6); Peroxisomal membrane signal receptor PTS1 (PEX5) promoter (one example of which is provided as SEQ ID NO:7); and catalase (CAT) promoter (one example of which is provided as SEQ ID NO: 9). As will be apparent to those of skill in the art, the promoter sequences provided in SEQ ID NOs: 1-12 are intended for illustrative purposes only and suitable variants of these may be used within the invention, depending on the host cell used.

The host cell may be a yeast cell, for example, selected from the group consisting of: *Pichia pastoris, Komagataella kurtzmanii, Komagataella phaffii, Pichia angusta, Pichia guillermordii, Pichia methanolica, Pichia inositovera, Hansenula polymorpha, Candida boidinii, and Yarrowia lipolytica.* In some embodiments, the yeast is *Pichia pastoris.*

In some embodiments, the nucleic acid molecule further comprises a secretion peptide in frame with the transgenic cell product of interest, preferably upstream in the direction of transcription and translation relative to the product of interest or gene of interest.

In some embodiments, the nucleic acid molecule further comprises an expression tag in frame with the transgenic cell product of interest, preferably at the C-terminus or N-terminus of the product of interest or gene of interest.

The suitable host cell culture density may be 250-350 g/L of culture (wet cell weight).

As discussed herein, in some embodiments of the invention, steps (c), (d) and (e) are repeated more than once. Specifically, especially when the transgenic cell product of interest includes a secretion sequence, the transgenic cell product of interest may be recovered from the growth media and additional non-repressing carbon source and inducer compound may be added to sustain growth of the host cell culture in batch phase so that product continue to be produced by the cells and recovered from the media.

In some embodiments, the host cell culture density is determined prior to adding the induction compound so that the inducer compound is added at a concentration that is sufficient to induce the inducible promoter at that host cell culture density.

In some embodiments, the non-repressing carbon source is initially added to the host cell culture in stages, for example, starting prior to exhaustion of the growth repressing carbon source so that initially the host cell culture is growing on both the repressing carbon source and the non-repressing carbon source. In some embodiments, the repressing carbon source is the major carbon source initially and the levels thereof are allowed to decrease until the non-repressing carbon source is the sole carbon source. As discussed herein, this prevents lags in growth of the host cell culture, as there is a gradual transition from the repressing carbon source to the non-repressing carbon source rather than an abrupt shift.

The non-repressing carbon source may be selected from the group consisting of sorbitol, mannitol, trehalose and alanine.

The fermentable repressing carbon source may be glycerol or glucose.

In some embodiments, the expression vector or recombinant vector comprises an origin of replication that enables the vector to propagate in, for example, *E. coli* for amplification and cloning purposes.

In some embodiments, the recombinant vector comprises selectable "marker genes", which enable the selection of host cells (both *E. coli* and yeast cells) transformed with a recombinant cassette of the application. Examples of selectable marker genes include but are by no means limited to genes encoding for proteins such as aminoglycoside 3'-phosphotransferase which confers resistance to G418 antibiotic, or hygromycin B phosphotransferase which confers resistance to hygromycin. Other suitable selectable marker genes will be readily apparent to one of skill in the art.

In some embodiments of the invention, the expression vector further comprises a secretion peptide (e.g. αMF) that is for example linked or fused or in frame with the transgenic cell product of interest so that when expression of the transgenic cell product of interest is driven by the inducible promoter, the peptide or polypeptide that is produced from the resulting transcript includes a secretion peptide which directs the nascent polypeptide chain to the secretion pathway, as discussed herein. In this manner, the product of the gene of interest (GOI) is operably linked downstream of the secretion peptide.

In some embodiments, the polypeptide produced by the expression vector further comprises, at the C' or N' terminal thereof, a detection tag that facilitates the detection of the protein of interest for example by means of Western Blotting. As will be known by those of skill in the art, human influenza hemagglutinin (HA) tag, Myc tag, FLAG tag or HIS tag are examples of short peptides that can be used as a detection tag.

As discussed herein, the repressing carbon source may be any fermentable carbon source, for example, but by no means limited to glycerol or glucose.

As will be apparent to those of skill in the art, as used herein, "batch phase" indicates intensive culture growth, for example, so that the host cell culture reaches high densities, e.g. 250-350 g/L of culture (wet cell weight).

As will be appreciated by one of skill in the art and as discussed herein, the specific density of the host cell culture when the carbon source is switched from a repressive carbon source to a non-repressive carbon source may vary, depending on the product being expressed and the desired outcome. As such, while a lower cell density will in theory produce less protein, this may be desirable if the product or protein being produced is for example toxic to the cell or otherwise problematic to synthesize and/or recover at higher densities. Similarly, while higher cell densities may not be healthy for the culture overall, in some embodiments, this higher density may be desirable for efficient production of the product.

As discussed herein, while not wishing to be bound to a particular theory or hypothesis, it is believed that the inducer compound is degraded by formate dehydrogenase.

As discussed herein, the inducer compound may be added and the transgenic cell product of interest recovered from the host cell culture multiple times, depending of course on the nature of the transgenic cell product being produced. As will be apparent to one of skill in the art, in these embodiments, it may be desirable to incorporate a secretion peptide into the nucleic acid molecule encoding the transgenic cell product to facilitate recovery and permit multiple "induction and recovery" stages. In these embodiments, non-repressing carbon source may also be fed, either continuously or in batches, to the host cell culture, as discussed herein.

The disclosure provides a method for producing added value products like polypeptides or small molecule compounds by the culturing of methylotrophic yeast without use of methanol as an inducer, that is, with the proviso that no methanol is added as an inducer. Instead, the method uses a non-repressing carbon source for feeding and an alternative inducer for expression of (a) gene(s) of interest. Accordingly, in one embodiment, provided herein is a process for producing added value compounds using methylotrophic yeast host expression system that comprises (a) nucleotide sequence(s) encoding the gene(s) of interest, comprising (i) culturing the yeast host cells in a batch phase providing a feeding for robust growth; and (ii) culturing the host expression system in a fed-batch phase providing a feeding with an alternative inducer, or (ii) culturing the host expression system in a continuous phase providing feeding in continuous fermentation regime with an alternative inducer.

The batch and fed-batch phases carbon source, can be any carbon source except methanol. In one embodiment, the first and/or the second sources comprise one or more of glycerol, alanine, lactate, glycerol, glucose, ethanol, citrate, sorbitol, xylose, trehalose, arabinose, fructose, melibiose, maltose, rhamnose, mannose, mannitol, and raffinose. In one embodiment, the batch phase carbon source is glycerol. In another embodiment, the fed-batch and induction phase carbon source is sorbitol. Among them glycose, glycerol, ethanol, citrate, xylose, arabinose, fructose, melibiose, maltose, rhamnose, mannose, and raffinose belong to repressing carbon sources; whereas sorbitol, mannitol, alanine and trehalose are non-repressing carbon sources.

The continuous and induction phase carbon source can be any non-repressing carbon source except methanol. In one embodiment, the first and/or the second sources comprise one or more of alanine, sorbitol, mannitol. In one embodiment, the induction phase carbon source is sorbitol.

In one embodiment, a promoter is a regulatory nucleotide sequence that drives expression of a gene of interest.

In one embodiment, an inducer is a compound that regulates gene expression.

In one embodiment, an inducer comprises one or more of formaldehyde, S-formylglutathione, S-hydroxymethyl glutathione, formic acid or any alkali metal or ammonium salt of formic acid or an alkaline earth metal salt of formic acid is used. Exemplary of such inducers are sodium formate, potassium formate, and ammonium formate.

In one embodiment, the regulatory sequence is a promoter. The promoter is a regulatory nucleotide sequence in the host cell or host expression system that drives the expression of a gene of interest. In another embodiment, the promoter is a constitutive promoter or an inducible promoter. In one embodiment, the promoter is selected from a group consisting of, the FDH promoter (NAD⁺-dependent formate dehydrogenase) promoter, the Alcohol oxidase 2 (AOX2) promoter, a dihydroxyacetone kinase (DAK) promoter, a Dihydroxyacetone synthase 1,2 (DAS1,2) promoter, the Formaldehyde dehydrogenase 1 (FLD1) promoter, the Fructose 1,6-bisphosphate aldolase (FBA) promoter, the Peroxisomal membrane signal receptor PTS1 (PEX5) promoter, and a Catalase (CAT) promoter.

In one embodiment, the media comprising the host expression system is oxygenated. In another embodiment, the batch phase feed is provided at a rate that maintains a specific growth rate (µ) of the host expression system in the culture to be in a range from about 0.03 h⁻¹ to about 0.5 h⁻¹. In another embodiment, the fed-batch phase or continuous feed is provided at a rate that maintains a specific growth rate (µ) of the host expression system in the culture to be in a range from about 0.0001 h⁻¹ to about 0.465 h⁻¹.

For illustrative purposes, it is of note that a very fast growth rate for the host cell, for example, growth during the batch phase, is considered to be about 0.3 - 0.4 H⁻¹, whereas a slow growth rate may be for example about 0.01 - 0.04 H⁻¹, which may be the growth rate of the cells during the induction phase. As will be appreciated by one of skill in the art, in some embodiments, the non-repressing carbon source is supplied to or present in the growth medium at a concentration or percentage that will support growth of the host cell culture at about 0.3 - 0.4 H⁻¹ while the non-repressing carbon source is supplied to or present in the growth medium at a concentration or percentage that will support growth of the host cell culture at about 0.01 - 0.04 H⁻¹.

In some embodiments, the batch phase and fed-batch /continuous phase are each carried out at a temperature of about 21°C to about 30°C. In one embodiment, the batch phase and fed-batch phase are each carried out at a temperature of about 25°C.

In one embodiment, the polypeptide is a heterologous polypeptide.

In some embodiments, the polypeptide comprises about ten or more amino acids.

The term "heterologous" refers to a polynucleotide, gene, polypeptide, or an enzyme not normally found in the host organism (*e.g*., recombinant cell). "Heterologous" also includes a native coding region, or portion thereof, that is reintroduced into the host organism in a form that is different from the corresponding native gene, *e.g.,* not in its natural location in the host's genome. The heterologous polynucleotide or gene may be introduced into the host organism by, *e.g.,* gene transfer, for example, by transformation or transfection. A heterologous gene may include a native coding region that is a portion of a chimeric gene including non-native regulatory regions that is reintroduced into the native host. Foreign genes can be conceptualized as native genes inserted into a non-native organism, or chimeric genes. Thus, "heterologous" polypeptides are those polypeptides foreign to the host cell being utilized, such as a plant or human protein being produced by yeast or bacteria. While the heterologous polypeptide may be prokaryotic or eukaryotic, in some embodiments it is eukaryotic. In some embodiments, it is a plant or human protein or peptide. In some embodiments, it is a polypeptide (*e.g*., enzyme).

Variants and/or fragments of the polypeptides described herein may also be prepared by the methods disclosed herein.

In some embodiments, activity of a polypeptide of the present invention, including activity of the variants and fragments thereof, can be determined by methods known in the art.

In one embodiment, the polypeptide is human epidermal growth factor (hEGF) comprising the amino acid sequence set forth in SEQ ID NO:17 (NCBI Accession No. XP_016863338.1) hEGF) is a ~6.2 kDa polypeptide composed of 53 amino acid residues with three intramolecular disulfide bonds. One of its maj or biological functions is to promote the generation of new epithelial and endothelial cells, and to stimulate tissue repairs. hEGF had been produced in various host systems including *Escherichia coli, Saccharomyces cerevisiae* and *baculovirus.* In *E. coli,* the produced hEGF tends to form inclusion bodies, which dramatically complicates downstream processes, because it requires laborious procedures of refolding and multistep purification.

In another embodiment, the polypeptide is an extracellular superoxide dismutase [Cu-Zn] (hSOD3) comprising the amino acid sequence set forth in SEQ ID NO:18 (NCBI Accession No. NP_003093.2) SOD is a ~30kDa polypeptide and reported to be a multimeric glycoprotein composed of at least four identical subunits in human extracellular fluids with heterogeneous affinity for heparin. The potential demand for SOD in human healthcare is growing up; therefore, production of biological active SOD is of a great interest. Production of therapeutic proteins by genetically engineered yeasts was shown to be a cost-effective alternative to tissue cultures or purification from animal tissues.

In another embodiment, the polypeptide is a human Lactoferrin (hLF) comprising the amino acid sequence set forth in SEQ ID NO:19 (NCBI Accession No. AAB60324.1) Lactoferrin (LF) is a member of the transferrin family of iron-binding glycoproteins. It was originally found in mammalian exocrine secretions and in specific granules of polymorphonuclear leukocytes.

In another embodiment, the polypeptide is the receptor binding domain (RBD) of S (spike) glycoprotein of SARS-CoV-2 virus comprising the amino acid sequence set forth in SEQ ID NO:20 (PDB: 7CM4_A). The surface exposed location of the S glycoprotein renders it a direct target for host immune responses, making it the major target of neutralizing antibodies. The S protein is considered to be a primary target for vaccine design as well as antiviral therapeutics.

A polypeptide prepared by the method of the present invention can be isolated after expression by techniques known in the art, including, but not limited to, affinity chromatography, ion-exchange chromatography, antibody affinity, size-exclusion, or any other method that eliminates a substantial portion of the culture and/or cellular debris from the polypeptide. In some embodiments, the process provides a substantially purified polypeptide. The isolated polypeptide can have activity similar to the corresponding native protein that it is derived from. The polypeptide can be isolated in a correctly folded state or conformation, approximating that of the native protein, or can be further renatured or modified to put it into a correctly folded conformation using a variety of methods and/or reagents known in the art.

In one embodiment, the host cells are *Pichia pastoris* (*e.g., Komagataella spp*), *Pichia angusta, Pichia guillermordii, Pichia methanolica,* or *Pichia inositovera.*

In some embodiments, the recombinant or host cell is *Pichia pastoris.*

In other embodiments, the recombinant or host cell is a Mut^{S} (methanol utilization slow) strain of *P. pastoris* KM71 and KM71h. It is of note however that Mut⁺ strains such as GS115 may be used within the invention and use of a Mut^{S} strain is not a requirement of the invention.

In another embodiment, the host cell or recombinant cell is *Hansenula polymorpha, Candida boidinii,* or *Yarrowia lipolytica.*

In one embodiment, a heterologous polynucleotide encoding the polypeptide is provided on a vector (*e.g*., plasmid) suitable for integration into the genome of the host cell in single or multiple copies per host cell. In some embodiments, the vector is a nucleotide sequence integrated into the genome.

In one embodiment, the vector is a eukaryotic expression vector, preferably a yeast expression vector.

In another embodiment, the expression vector is a cloned recombinant nucleotide sequence, such as the DNA sequence required for transcription of one or more recombinant gene(s) or peptides of interest and their mRNA translation in appropriate host organisms.

In other embodiments, such expression vectors typically include one or more of an origin for autologous replication in a host cell, an appropriate marker (*e.g*., gene that confers resistance to antibiotics such as zeocin, kanamycin G418 or hygromycin), a restriction enzyme cleavage site, an appropriate promoter sequence and a transcription terminator, and these components are operably linked to interact with each other.

In some embodiments, expression vectors include, but are not limited to, cloning vectors, modified cloning vectors, and specifically designated plasmids. The expression vector of the present invention may be any expression vector suitable for expression of a recombinant gene in a host cell, which is selected according to the host organism.

In other embodiments, regulatory sequences include: a transcriptional promoter and enhancer or RNA polymerase binding sequence, a ribosomal binding sequence, including a translation initiation signal. Additionally, depending on the host cell chosen and the vector employed, other sequences, such as an origin of replication, additional DNA restriction sites, enhancers, and sequences conferring inducibility of transcription may be incorporated into the expression vector.

In some embodiments, the nucleotide sequence of the gene of interest is under the control of a constitutive promoter, a promoter responsive to a carbon source which is fed during a batch phase of culturing, and/or a promoter responsive to a carbon source and to an inducer, which is fed /added during a fed-batch phase of culturing

In one embodiment, the promoter is an endogenous promoter, wherein the polynucleotide encoding the polypeptide is integrated into the genome of the yeast host cell such that the endogenous promoter is operably linked to the heterologous polynucleotide, thereby capable of driving its expression.

In other embodiments, the expression vector pL (SEQ ID NO:3) further comprises a secretory leader sequence effective for inducing the secretion of polypeptide from the host cell.

In other embodiments, the expression vector pL (SEQ ID NO: 13) further comprises the HA-tag (Hemagglutinin tag) for the routine detection by Western Blot using anti-HA-tag antibodies.

In some embodiments, the secretory leader sequence may originate from a yeast α-factor source, for example from αMF , yeast phosphatase (PHO), heat shock proteins (HSP), as well as HSP tag repeats, invertase (SUC2) tag, OST1 tag, DDDK tag or combination thereof, or any other secretion peptide described in the literature.

In some embodiments, the step of growing the recombinant cell comprising the heterologous polynucleotide includes growing the cell in a medium comprising a first carbon source, for example, a fermentable or repressing carbon source.

Examples of other ingredients that may be added to the medium are discussed herein; however, suitable ingredients and the amounts thereof will be readily apparent to those of skill in the art and/or may be determined through routine experimentation.

In one embodiment, the medium is an aqueous medium comprising the first carbon source, and optionally one or more further ingredients such as, for example, salts (e.g., phosphate and/or sulphate, and the like), antibiotics, vitamins, trace metal ions, agents to keep the pH at a desired level, phosphate salts, and/or antifoaming agents.

In another embodiment, the medium comprises one or more of phosphoric acid, calcium sulfate, potassium sulfate, magnesium sulfate, potassium hydroxide, and glycerol.

In some embodiments, the medium further comprises one or more of cupric sulfate, sodium iodide, manganese sulfate, sodium molybdate, boric acid, cobalt chloride, zinc chloride, ferrous sulfate, biotin, and sulfuric acid.

In other embodiments, the batch and fed-batch carbon source (or first carbon source) comprises one or more of alanine, lactate, glycerol, glucose, ethanol, citrate, sorbitol, xylose, trehalose, arabinose, fructose, melibiose, maltose, rhamnose, mannose, mannitol, and raffinose.

In one embodiment, the continuous and induction phases carbon source (or second carbon source or non-repressing carbon source) comprises one or more of alanine, sorbitol, mannitol, and trehalose.

Specifically, whereas the conventional yeast (*S*. *cerevisiae*) prefers glucose or its precursors (disaccharides) so that to assimilate it through glycolysis, methylotrophic yeast like *Pichia pastoris* prefer glycerol as a carbon source assimilating it through G3P (glycerol-3-phosphate) - DHAP (dihydroxyacetone phosphate) pathway. However, *Pichia* cultures can readily use glucose as well. Considering that glycerol is cheaper, most culturing processes are designed for glycerol use in batch phase
In some embodiments, the batch phase and/or the fed-batch carbon source are non-fermentable carbon sources.

For example, in one embodiment, in a batch phase, the recombinant cell is cultured in a saline medium with a glycerol.

For example, is some embodiments, the recombinant cell comprising the heterologous polynucleotide is grown in the medium in a fermenter, which, as used herein, also refers to for example a bioreactor or any other suitable apparatus for culturing the recombinant cells) employing a batch protocol whereby the cells are grown using the first carbon source (*e.g*., glycerol). Cell growth may be monitored periodically and may continue until the first carbon source (*e.g.,* glycerol) is consumed. In some embodiments, complete consumption of the first carbon source (*e.g.,* glycerol) is indicated by a spike in dissolved oxygen (DO) levels to 100%. The length of time needed to consume all the first carbon source (*e.g.,* glycerol) can vary depending on the density of the initial inoculum. That is, addition glycerol or other fermentable carbon source may be added in order to bring the host cell culture density to the desired density.

In some embodiments, sampling of the culture to measure cell density may be performed at the end of the first carbon source (*e.g.,* glycerol) feed stage, *e.g.,* cell density can be measured by withdrawing a sample from *e.g.,* the fermenter at each timepoint and using an aliquot for measuring cell density *e.g.,* at a wavelength of 600 nm. In other embodiments, cell growth can be evaluated by measuring the wet cell weight, pH, microscopic purity, protein concentrations and/or activity.

In some embodiments, the step of growing comprises adding a culture comprising the cell to the medium comprising the first carbon source.

In another embodiment, an initial amount of the first carbon source in the medium is at about 4% by volume of the first carbon source.

In other embodiments, after consumption of glycerol (*e.g.,* at completion of the batch phase), a carbon source-limited (*e.g.,* glycerol-limited) feeding phase (*e.g.,* employing a fed-batch protocol) follows *e.g.,* until the desired level of biomass is reached. In other embodiments, after consumption of glycerol (*e.g.,* at completion of the batch phase), a glycerol-limited feeding phase commences until a desired level of the biomass is reached.

Thus, in some embodiments, once the first-carbon source (*e.g.,* glycerol) is depleted during the batch phase (*e.g.,* glycerol batch phase), a second phase (*e.g.,* glycerol fed-batch phase) is begun by adding the appropriate carbon source (*e.g.,* glycerol) to the medium at a limiting growth rate of the recombinant cells.

For example, in one embodiment, the step of growing further comprises continuously adding the first carbon source to the medium at a first feed rate from a solution comprising the first carbon source.

In another embodiment, the feed rate of the fed-batch carbon source is provided at such a rate so to maintain the specific growth rate (µ) of the culture in the range 0.001 - 0.5 h⁻¹.

In another embodiment, the first feed rate is initiated after the initial amount of the batch phase carbon source is completely consumed by the culture.

In other embodiments, the step of culturing comprises adding the fed-batch carbon source to the medium at a second feed rate and decreasing the first feed rate. As discussed herein, this provides a gradual transition from growth of the host cell culture on the repressing carbon source to growth on the non-repressing carbon source.

In another embodiment, the feed rate of the continuous and/or induction phase carbon source is provided at such a rate so to maintain the specific growth rate (µ) of the culture in the range 0,001 - 0,5 h⁻¹.

In other embodiments, once the transition from the first carbon source feed to the second carbon source feed is completed, an aqueous solution comprising the second carbon source and trace salts is introduced into the medium.

In one embodiment, the second carbon source (*e.g.,* sorbitol) feed is stopped if DO cannot be maintained above 20%, then resumed when the DO increases to at least about 20%. For example, increasing agitation, aeration, pressure and/or oxygen feeding can help increase and/or maintain the DO above 20%. Generally, culturing of methylotrophic yeast is carried out under aerobic conditions, so the cells are respiratory active on either of the substrates. Gradual increase of DO means that the culture has not adapted to the new carbon source yet and not actively consuming it. So, adding more of the second carbon source when DO is not stabilized yet and still is in uptrend can lead to accumulation of the substrate to a stressful threshold concentration. As will be appreciated by one of skill in the art, the repressing carbon source used in batch and fed-batch phases needs to be completely depleted for efficient inducing of the listed promoters to be obtained upon adding an inducing agent.

In one embodiment, the inducer comprises one or more of formaldehyde, S-formylglutathione, S-hydroxymethyl glutathione, formic acid or any alkali metal salt of formic acid or an alkaline earth metal salt of formic acid.

In another embodiment, the inducer comprises sodium formate, potassium formate, and/or ammonium formate.

In some embodiments an inducer is added by doses or boluses in amount of 0.001-2.0g per 1 L of the culture up to 20 times a day.

While not wishing to be bound to a particular theory or hypothesis, when the formates or formic acid is added, it is apparently degraded by NAD⁺ dependent formate dehydrogenase enzyme (FDH) produced by *Pichia pastoris* cells intracellularly, so it is depleted in a while. It is not well known how fast it is dissimilated but there is an increase in yield in some cases when it is added it up to 3-4 times a day.

For example, in one embodiment, during the induction phase the 50%(w/v) solution of potassium formate is added in amount of 1g/1L of the culture 2 times a day.

In one embodiment, the method of the present invention allows the production of a heterologous polypeptide or any other added value compound without methanol or with the proviso that no methanol or substantially no methanol, that is, insufficient methanol on its own, is added.

In some embodiments, the first and/or the second carbon sources can be any carbon source except methanol. In one embodiment, the first and/or the second sources comprise a compound selected from the group consisting of alanine, lactate, glycerol, glucose, ethanol, citrate, sorbitol, xylose, trehalose, arabinose, fructose, melibiose, maltose, rhamnose, mannose and raffinose. In one embodiment, the batch and fed-batch phases carbon source is glycerol and the induction phase carbon source is sorbitol.

The invention will be further described in the following examples, which do not limit the scope of the invention described in the claims.

### EXAMPLES

### Example 1

### Assessment the induction potential or strength of the set of promoters from Pichia pastoris (Komagataella phaffii) strains KM71h and GS115.

The induction pattern of the following promoters were assessed by the measuring the transcription level by means RT-qPCR method: FDH promoter (NAD⁺-dependent formate dehydrogenase) promoter, the Alcohol oxidase 1 (AOX1 ) promoter, the dihydroxyacetone kinase (DAK) promoter, the dihydroxyacetone synthase 2 (DAS2 ) promoter, the Formyl-glutathione dehydrogenase (FGH) promoter (not according to the invention), the Fructose 1,6-bisphosphate aldolase (FBA) promoter, the Peroxisomal membrane signal receptor PTS1 (PEX5) promoter, the Alcohol dehydrogenase 2 (ADH2) promoter (not according to the invention). Constitutive GAP (Glyceraldehydes-3-phosphate dehydrogenase) promoter's induction level was used as a reference.

The yeast cultures *Pichia pastoris* GS115 and KM71h strains were grown under repressed, derepressed and induced conditions. The repressed conditions suggest a repressive carbon source in a media, which makes the involved promoters repressed, for example glycerol. The derepressed conditions suggest a non-repressive carbon source in the media, for example sorbitol or any depleted carbon source, which switches the involved promoter to derepressed state. Induced conditions suggest the addition of a compound inti the media, called an inducer, which makes the involved promoters induced.

The culture of *P. pastoris* strain GS115 was inoculated from YPD plate into the shacking flask with 10ml of liquid YPD. The culture was growth overnight and reinoculated in a following way:
- 1% of inoculum to three flasks with 10ml of YN media (+histidine) + 1% (v/v) glycerol, which represents repressed conditions
- 1% of inoculum to three flasks with 10ml of YN media (+histidine) + 1% (w/v) D(+) sorbitol, which represents derepressed conditions
- 1% of inoculum to three flasks with 10ml of YN media (+histidine) + 1% (w/v) D(+) sorbitol + 0.2 % (v/v) methanol, which represents induced conditions
- 1% of inoculum to three flasks with 10ml of YN media (+histidine) + 1% (w/v) D(+) sorbitol +1 % (w/v) potassium formate, which represents induced conditions

Those 4 media represent repressed, derepressed and induced (both with methanol and formate) conditions. The cultures were incubated in a temperature-controlled orbital shaker at 29⁰C, 250RPM for 6 hours followed by harvesting the biomass. Total RNA was extracted with RNeasy kit (Qiagen, Germany) according to the manufacturer's protocol. Reverse transcription was done with the High-Capacity cDNA Reverse Transcription Kit (ThermoFisher Scientific, USA). Specific primers for each appropriate gene: FDH, FGH, DAK, DAS2, AOX1 , FBA, PEX5 and ADH2 were used for RT-PCR to assess the level of their transcription and thus the induction level of the respective promoter. The SYBR green method was used for RT-PCR, with the SYBR^{®} Green qPCR master mix (Bio-Rad, USA) according to the manufacturer's protocol.

The ΔCt method was used for building standard curves. GAP gene (D-glyceraldehyde 3'-phosphate dehydrogenase) was used as a standard reference gene.

### List of the primers used for RT-PCR

qGAP_for; CTGGTGTCGACTACGTCATTGAGTC (SEQ ID NO:22)
qGAP_rev; GCATTGGAGACAATGTTCAAGTCAG (SEQ ID NO:23)
qFDH_for; ACTCCATTCCATCCAGCCTACATC (SEQ ID NO:24)
qFDH rev; CATAACGACATGCTCAGCCACTG (SEQ ID NO:25)
qFGH for; CTTCAACACAAGTCCGATGAGACG (SEQ ID NO: 26)
qFGH_rev; GGTTGCCAAAATGCCTTCTCTG (SEQ ID NO:27)
qDAS2_for; GGCCAAGTACGGTTTCGATGTC (SEQ ID NO:28)
qDAS2_rev; CCTCTAATACGGGCCTTTAATTCCTCA (SEQ ID NO:29)
qDAK for; AGGACACGAGCCTCTACATGCTG (SEQ ID NO:30)
qDAK_rev; GGCAAGACCGAAGTGAAGAATGTC (SEQ ID NO:31)
   qAOX1 for; GTGAGCACACTGAGACCACATGG (SEQ ID NO:32)
qAOX1_rev; GAGCGGTGGTGTAGGTGTTACAAC (SEQ ID NO:33)
qFBA2 for; AAAAGCGGTGTCATCGTTGGAG (SEQ ID NO:34)
qFBA2 rev; TGGAAAAAAGCAGCACCTCCC (SEQ ID NO:35)
qPEX5_for; ACACAACATGACACATCCTTGCAAC (SEQ ID NO:36)
qPEX5_rev; TGCATTACGTTCAGCTCGTGTTG (SEQ ID NO:37)
qADH2 for; AAGGGTGACTGGCCATTGGAC (SEQ ID NO:3 8)
qADH2 rev; TTGGCACAACTGGATTCAGCAC (SEQ ID NO:39)

The results are shown in the Fig. 1
YN medium (+histidine): 6.7g of Yeast Nitrogen Base with Ammonium sulphate; 20mg L-histidine; bring to 1 l with distilled water.

### Example 2

### Cloning hEGF, hSOD3 and hLF to pL integration vector, transformation into yeast strain and screening

The codon optimized genes of hEGF (SEQ ID NO:14), hSOD3 (SEQ ID NO:15),hLF (SEQ ID 16) and RBD (SEQ ID NO:21) were cloned to the standard plasmid pL_FDH with the NheI and PacI cloning sites, yielding the plasmids pL hEGF, pL_hSOD3 and pL_hLF respectively, where the GOI (gene of interest) - hEGF or hSOD3 or hLF or RBD were fused with αMF secretion tag downstream of the inducible FDH promoter but upstream of the GOI. The plasmids were linearized with Bsp119I restriction site and transformed into GS1 15 strain of *Pichia pastoris* by electroporation, using BTX electroporation (BTX, USA) according to the manufacturer's protocol. **Fig. 2** schematically illustrates the construct containing the GOI and its integration within the yeast genome. Resistance to G418 antibiotic was used for the selection of the transformed clones. Thus, after the transformation the yeast culture was plated onto YPD plates with G418 (0.8mg/ml). The integration of the respective GOI was verified with PCR using forward primer annealing to 3' of pAOX1 and reverse primer annealing to 5' of the GOI.

At least 10 clones were grown in the shaking flasks. Selected clones of the recombinant strains GS115/pL_hEGF, GS115/pL_hSOD3, GS115/pL_hLF and GS115/pL_RBD were inoculated into 10 ml of BMGY medium, incubated in temperature controlled orbital shaker for 24 hours at 29⁰C, 270 RPM. Pre-grown culture was then spun down at 2000g, washed in distilled water divided into two aliquots. Each aliquot was re-inoculated into 10 ml of BMFSY medium, incubated for another 72 hours at 29⁰C, 270 RPM in the shaking flasks under inducible conditions. After 24-hours the cultures were induced for 3 consecutive days with a daily dose of the final concentration of 0,2%(w/v) potassium formate + 1%(w/v) sorbitol.
YPD liquid: 10g of Yeast extract; 20g of Peptone; 20g Dextrose; bring to 1 L with distilled water.
YPD agar: 10g of Yeast extract; 20g of Peptone; 20g Dextrose; 20g Agar; bring to 1 L with distilled water.
BMGY: 10g of Yeast extract; 20g of Peptone; 100 ml of 1 mM of Potassium phosphate buffer; 6.7g of Yeast Nitrogen Base with Ammonium sulphate; 10g of Glycerol; bring to 1 L with distilled water.
BMSFY: 10g of Yeast extract; 20g of Peptone; 100 ml of 1 mM of Potassium phosphate buffer pH 6.0; 6,7g of Yeast Nitrogen Base with Ammonium sulphate; 20g of Sorbitol; 0,5g of Potassium formate; bring to 1 l with distilled water.

### Example 3

### Fermentation (Culturing) the selected clones of the following recombinant strain GS115/pL_hEGF, GS115/pL_hSOD3, GS115/pL_hLF

The recombinant strains of *Pichia pastoris* GS115/pL_hEGF, GS115/pL_hSOD3, GS115/pL_hLF were used to carry out the fermentation to produce hEGF, hSOD3 and hLF respectively. Fermentation was carried out in a temperature-controlled fermenter (10L working volume) to maintain the temperature at 28⁰C. The pH of the medium throughout the fermentation was controlled automatically using a pH probe, a controller (New Brunswick, BioFlo 3000) and a computer with Biocommand batch software (Eppendorf AG) was used to monitor and control the fermentation.

Fermentation was carried out using a cell culture medium comprising reduced basal salts medium (BSM) with PTM1 trace salts and kanamycin (50mg/L optionally). The ingredients (per 1 liter) of BSM are listed in Table 2.

The culturing or each strain was carried out at 25°C and a dissolved oxygen (DO) content in the medium at a level of 20% or higher. The pH during fermentation was maintained at 6.5 for secreting protein into the medium and for optimal growth by titrating a solution of ammonium hydroxide into the fermentation vessel. The agitation rate was maintained in the range from about 500 rpm to about 1000 rpm to maintain the above-mentioned oxygen concentration in the medium. Aeration rate was carried out to provide about 0.1 to 1.0 volume of oxygen (in liters) per volume of fermentation culture (in liters) per minute (vvm), so as to maintain the above-mentioned dissolved oxygen concentration (DO) in the medium. A minimum amount of Antifoam A (cat. no. A5633) or Antifoam 204 (cat. no. A6426) (Sigma-Aldrich, St. Louis, MO) was used to avoid excess foaming which can cause denaturation of secreted protein and can contribute to reducing the headspace in the fermenter. For the carbon sources, glycerol and sorbitol were used at variable rates as the first carbon source and the second carbon source respectively. Here, glycerol was used as the first carbon source to accumulate cell mass and sorbitol was used as the second carbon source to sustain cell growth and for inducing protein expression.

**Table 1. Reduced Basal Salts Medium (BSM)**

| | |
|---|---|
| Phosphoric acid, 85% | 4.25 ml |
| Calcium sulfate | 0.136 g |
| Potassium sulfate | 2.86 g |
| Magnesium sulfate | 1.13 g |
| Potassium hydroxide | 0.64 g |
| Glycerol | 40 g |
| Water | bring the volume to 1 L |

Twelve (12) ml of filter-sterilized PTM1 trace salts was added to 1 L of BSM medium. The ingredients of PTM1 trace salts (per liter) are listed in Table 3.

**Table 2. PTM1 trace salts (per 1 liter)**

| | |
|---|---|
| Cupric sulfate-5H2O | 6.0 g |
| Sodium iodide | 0.08 g |
| Manganese sulfate-H2O | 3.0 g |
| Sodium molybdate-2H2O | 0.2 g |
| Boric Acid | 0.02 g |
| Cobalt chloride | 0.5 g |
| Zinc chloride | 20.0 g |
| Ferrous sulfate-7H2O | 65.0 g |
| Biotin | 0.2 g |
| Sulfuric Acid | 5.0 ml |
| Water to a final volume of 1 liter | |

Cell growth was monitored at various time points during fermentation by measuring the optical density (OD) of the culture at a wavelength of 600 nm (OD₆₀₀) and by measuring the wet cell weight. The metabolic rate of the culture was monitored by monitoring dissolved oxygen (DO) and changes in the concentration of dissolved oxygen in response to carbon availability.

### Measurement of Dissolved Oxygen ("DO ")

The second carbon source feeding rate was adjusted in response to DO levels.

The level of the carbon source in the culture is an important determinant for protein induction. For example, changes in the DO concentrations (DO spikes) can be used to determine whether all the glycerol is consumed from the culture before adding the second carbon source e.g., sorbitol. Monitoring the level of carbon source ensures that the sorbitol feed does not over accumulate in the fermenter.

### Fermenter Preparation and Glycerol Batch Phase

Fermentation started with the preparation of a seed culture flask that was used as an inoculum. Typically, a flask containing a total of 5 mL of BMGY media was inoculated with 50 uL of glycerol stock of either GS115/pL_hEGF or GS115/pL_hSOD3 or GS115/pL_hLF. The inoculate was grown at 29 °C, by shaking the flask at 250-300 rpm for 16-24 hours, until the optical density of the culture at 600nm (OD₆₀₀) was 2-6. This initial culture was sub-cultured into a second flask containing 5 mL BMGY media for an additional 24 hrs. On day 3, the second flask was sub-cultured into a 2-liter flask containing 200 mL BMGY media for another 16-24 hours, or until the OD₆₀₀ of the culture was 2-6.

This 200 mL culture served as the inoculum for the fermenter. A fermenter containing 4L of BSM was sterilized prior to inoculation with the yeast culture. After sterilization, the medium was cooled, and the temperature set to 28°C. DO and pH probes were calibrated according to the manufacturer protocol (Mettler Toledo< Germany). The medium was agitated with the Rushton impeller at 500RPM and higher and aerated at 1.0 vvm using compressed air to bring the DO of the medium to levels suitable for fermentation. The pH of the medium was adjusted to 6.5 using ammonium hydroxide prior to inoculation, followed by the aseptic addition of 4.35 ml of PTM1 trace salts per liter of fermentation medium. To avoid bacterial contamination, kanamycin was added to the medium at a final concentration of 100 ug/ml. This medium was inoculated using 200 mL of yeast culture of OD₆₀₀ at 5.0-6.0. The DO of the culture (medium + yeast cells) in the fermenter was measured following inoculation and was recorded as nearly100%. After the fermentation started, DO was monitored and controlled by the controlling unit of the fermenter using PID (Proportional-Integrative-Derivative) algorithm. If the DO level of the culture dropped below 20%, agitation was increased to bring the DO level of the culture above 20%. pH was also monitored and controlled by the controlling unit of the fermenter using PID (Proportional-Integrative-Derivative) algorithm, and adjusted by titrating the culture with the 30% (v/v) solution of ammonium hydroxide by the controlling unit.

Complete consumption of added glycerol was indicated by a spike in DO levels to 100%. The length of time needed to consume all the glycerol can vary depending on the density of the initial inoculum.

Sampling of the culture to measure cell density and wet cell weight was performed at the end of the glycerol feed stage and later at least twice daily. Cell density was measured by withdrawing a 5 ml sample from the fermenter at each time point and using a 1 ml aliquot for measuring cell density at a wavelength of 600 nm. Additionally, cell growth was evaluated by measuring the wet cell weight, pH, microscopic purity, protein concentrations and activity.

The wet cell weight at this stage, after the glycerol fed-batch stage was in the range from about 90 g/liter to about 150 g/liter.

### Glycerol fed-batch phase

Once the glycerol provided in the fed-batch phase was consumed, cell biomass was further increased by initiating a steady feed of glycerol. The glycerol feed was initiated using a 50% w/v solution of glycerol containing 12 ml PTM1 trace salts per liter of glycerol. The feed rate was set to 18.15 ml/hr /liter of the initial fermentation volume. Glycerol feeding was carried out for about four hours or longer (see below), until the wet cell weight was about 300 g/liter. The level of expressed protein was found to depend in part on the wet cell weight of the cell pellet from the glycerol feeding stage of fermentation. The length of the glycerol feeding phase, therefore, was varied to optimize protein yield.

### Transition Phase and Sorbitol Fed-Batch Phase

Once the wet cell weight of the culture during the glycerol feeding phase was about 250 g/liter to about 300 g/liter, the carbon source was switched from a first carbon source, glycerol, to a second carbon source, sorbitol. Such a transition from glycerol to sorbitol was carried out using a "mixed feed" of glycerol and sorbitol initially. During the mixed feed phase, glycerol feeding was slowly decreased from a rate of 18.15 ml/hr/L of culture medium to 0 ml/hr/L of culture medium over a period of 2 hours and the sorbitol feeding rate is slowly increased from 0 ml/hr/L of culture medium to 2.57 ml/hr/L of culture medium over the same 2 hours. The transition from a glycerol feed to a sorbitol feed was carried out at a rate that did not cause any significant spikes or drifts in the pH of the culture. Other surrogate measures of cell growth and cell health were also monitored during the transition phase.

Once the transition from a glycerol feed to a sorbitol feed was completed, induction of protein expression was initiated by introducing an aqueous solution of 50% sorbitol containing 12 ml PTM1 trace salts per liter of this sorbitol solution.

Increasing agitation up to 1000 RPM and pure oxygen was used to maintain the DO of the culture within a range of 20-25%. When the culture was fully adapted to sorbitol utilization (2-4 hours), the DO reading remained steady. After 2-4 hours at the 2.57 ml/hr/liter feed rate, the sorbitol feed rate was increased to about 5.13 ml/hr per liter initial fermentation volume. Then feed rate was adjusted once a day proportionally to the increase of biomass during the course of the fermentation.

Once the culture had adapted to sorbitol as the carbon source, the inducer (50% (w/v) solution of potassium formate) was added to the cell culture medium at the amount of 2ml/L of culture medium every 12 hours.

The entire sorbitol fed-batch phase lasted approximately 72 hours with a total of approximately 0.75 L sorbitol fed per liter of initial volume. The cell density increased during the sorbitol fed-batch phase to a final level of 350 to 500 g/liter wet cells.

SDS-PAGE and Western Blot was used to visualize the hEGF or hSOD3 or hLF using harvested samples of the supernatant of the culture media at the end of fermentation.

### Example 4

### Analysis of the samples with SDS-PAGE and Western Blot with anti HA-antibodies

The expression construct, which was integrated to the production strain genome, contained GOI fused with HA-tag at its C-terminus for detection and visualizing purposes and cloned downstream of the promoter FDH. Once either of hEGF, hSOD3, hLF or RBD was expressed and secreted into the media during fermentation, it could then be detected on Western blot with anti-HA-tag antibodies. The strain engineering including molecular cloning, transformation, PCR selection was done according to the standard protocols (Molecular Cloning: A Laboratory Manual, 3rd Edition, Cold Spring Harbor Laboratory Press, 2001).

The supernatant was harvested by centrifugation (10,000g for 5 min) of the vials containing an aliquot of the culture. SDS-PAGE was done with the 10ul of supernatant followed by the wet transfer to the PVDF membrane according to standard manufacturer's protocol (BioRad, USA). The membrane was then incubated with first anti HA-antibodies (cat. no sc-7392, Santa Cruz Biotechnology, USA) and then with anti-mouse goat antibodies (cat. No. G-21040, Invitrogen, USA) according to the standard protocol (Invitrogen, USA). The membrane was stained with the Pierce^{™} ECL Plus Western Blotting Substrate kit (cat. No. 32132, ThermoFisher Scientific, USA). The supernatant from a methanol-induced cultures was used as a reference signal (FIG. 3-6).

**Table 3.**

| **Nucleotide SEQ ID No.** | **Corresponding Amino acids SEQ ID No.** | **Accessory Element** |
|---|---|---|
| SEQ ID No. 1 | n/a | Promoter FDH (NAD⁺-dependent formate dehydrogenase) |
| SEQ ID No. 2 | n/a | Promoter AOX2 (alcohol oxidase 2) |
| SEQ ID No. 3 | n/a | Promoter PEX14 (peroxin Pex14p) |
| SEQ ID No. 4 | n/a | Promoter FGH (S-hydroxymethyl-glutathione hydrolase) |
| SEQ ID No. 5 | n/a | Promoter DAK (dihydroxyacetone kinase) |
| SEQ ID No. 6 | n/a | Promoter FBA2 (fructose 1,6-bisphosphate aldolase) |
| SEQ ID No. 7 | n/a | Promoter PEX5 (peroxisomal membrane signal receptor PTS1) |
| SEQ ID No. 8 | n/a | Promoter ADH2 (alcohol dehydrogenase 2) |
| SEQ ID No. 9 | n/a | Promoter CAT1 ( catalase) |
| SEQ ID No. 10 | n/a | Promoter DAS1 (Dihydroxyacetone synthase 1) |
| SEQ ID No. 11 | n/a | Promoter DAS2 (Dihydroxyacetone synthase 2) |
| SEQ ID No. 12 | n/a | Promoter FLD1(Formaldehyde dehydrogenase) |
| SEQ ID No. 13 | n/a | Plasmid µL |
| SEQ ID No. 14 | SEQ ID No. 17 | Gene/Protein hEGF, human epidermal growth factor |
| SEQ ID No. 15 | SEQ ID No. 18 | Gene/Protein hSOD3 extracellular superoxide dismutase [Cu-Zn] |
| SEQ ID No. 16 | SEQ ID No. 19 | Gene/Protein hLF human Lactoferrin |
| SEQ ID No. 20 | SEQ ID No. 21 | Gene/Protein RBD (Receptor Binding Domain of S (spike) protein of SARS-CoV-2 virus |

### Nucleotide sequences

SEQ ID NO:1 
ATTCTGGCCCTTGCACCTGATCGCGAAGGTGGAAATGGCAGAAGGATCAGCCTGGACGAAGCAACCAGTTCCAACTG
CTAAGTAAAGAAGATGCTAGACGAAGGAGACTTCAGAGGTGAAAAGTTTGCAAGAAGAGAGCTGCGGGAAATAAATT
TTCAATTTAAGGACTTGAGTGCGTCCATATTCGTGTACGTGTCCAACTGTTTTCCATTACCTAAGAAAAACATAAAG
ATTAAAAAGATAAACCCAATCGGGAAACTTTAGCGTGCCGTTTCGGATTCCGAAAAACTTTTGGAGCGCCAGATGAC
TATGGAAAGAGGAGTGTACCAAAATGGCAAGTCGGGGGCTACTCACCGGATAGCCAATACATTCTCTAGGAACCAGG
GATGAATCCAGGTTTTTGTTGTCACGGTAGGTCAAGCATTCACTTCTTAGGAATATCTCGTTGAAAGCTACTTGAAA
TCCCATTGGGTGCGGAACCAGCTTCTAATTAAATAGTTCGATGATGTTCTCTAAGTGGGACTCTACGGCTCAAACTT
CTACACAGCATCATCTTAGTAGTCCCTTCCCAAAACACCATTCTAGGTTTCGGAACGTAACGAAACAATGTTCCTCT
CTTCACATTGGGCCGTTACTCTAGCCTTCCGAAGAACCAATAAAAGGGACCGGCTGAAACGGGTGTGGAAACTCCTG
TCCAGTTTATGGCAAAGGCTACAGAAATCCCAATCTTGTCGGGATGTTGCTCCTCCCAAACGCCATATTGTACTGCA
GTTGGTGCGCATTTTAGGGAAAATTTACCCCAGATGTCCTGATTTTCGAGGGCTACCCCCAACTCCCTGTGCTTATA
CTTAGTCTAATTCTATTCAGTGTGCTGACCTACACGTAATGATGTCGTAACCCAGTTAAATGGCCGAAAAACTATTT
AAGTAAGTTTATTTCTCCTCCAGATGAGACTCTCCTTCTTTTCTCCGCTAGTTATCAAACTATAAACCTATTTTACC
TCAAATACCTCCAACATCACCCACTTAAACA
SEQ ID NO:2 
TTATTTTTTGACCGAATTCTTTTTTTCAGACCATATGACCGGTCCATCTTCTACGGGGGGATTATCTATGCTTTGAC
CTCTATCTTGATTCTTTTATGATTCAAATCACTTTTACGTTATTTATTACTTACTGGTTATTTACTTAGCGCCTTTT
CTGAAAAACATTTACTAAAAATCATACATCGGCACTCTCAAACACGACAGATTGTGATCAAGAAGCAGAGACAATCA
CCACTAAGGTTGCACATTTGAGCCAGTAGGCTCCTAATAGAGGTTCGATACTTATTTTGATAATACGACATATTGTC
TTACCTCTGAATGTGTCAATACTCTCTCGTTCTTCGTCTCGTCAGCTAAAAATATAACACTTCGAGTAAGATACGCC
CAATTGAAGGCTACGAGATACCAGACTATCACTAGTAGAACTTTGACATCTGCTAAAGCAGATCAAATATCCATTTA
TCCAGAATCAATTACCTTCCTTTAGCTTGTCGAAGGCATGAAAAAGCTACATGAAAATCCCCATCCTTGAAGTTTTG
 
TCAGCTTAAAGGACTCCATTTCCTAAAATTTCAAGCAGTCCTCTCAACTAAATTTTTTTCCATTCCTCTGCACCCAG
CCCTCTTCATCAACCGTCCAGCCTTCTCAAAAGTCCAATGTAAGTAGCCTGCAAATTCAGGTTACAACCCCTCAATT
TTCCATCCAAGGGCGATCCTTACAAAGTTAATATCGAACAGCAGAGACTAAGCGAGTCATCATCACCACCCAACGAT
GGTGAAAAACTTTAAGCATAGATTGATGGAGGGTGTATGGCACTTGGCGGCTGCATTAGAGTTTGAAACTATGGGGT
AATACATCACATCCGGAACTGATCCGACTCCGAGATCATATGCAAAGCACGTGATGTACCCCGTAAACTGCTCGGAT
TATCGTTGCAATTCATCGTCTTAAACAGTACAAGAAACTTTATTCATGGGTCATTGGACTCTGATGAGGGGCACATT
TCCCCAATGATTTTTTGGGAAAGAAAGCCGTAAGAGGACAGTTAAGCGAAAGAGACAAGACAACGAACAGCAAAAGT
GACAGCTGTCAGCTACCTAGTGGACAGTTGGGAGTTTCCAATTGGTTGGTTTTGAATTTTTACCCATGTTGAGTTGT
CCTTGCTTCTCCTTGCAAACAATGCAAGTTGATAAGACATCACCTTCCAAGATAGGCTATTTTTGTCGCATAAATTT
TTGTCTCGGAGTGAAAACCCCTTTTATGTGAACAGATTACAGAAGCGTCCTACCCTTCACCGGTTGAGATGGGGAGA
AAATTAAGCGATGAGGAGACGATTATTGGTATAAAAGAAGCAACCAAAATCCCTTATTGTCCTTTTCTGATCAGCAT
CAAAGAATATTGTCTTAAAACGGGCTTTTAACTACATTGTTCTTACACATTGCAAACCTCTTCCTTCTATTTCGGAT
CAACTGTATTGACTACATTGATCTTTTTTAACGAAGTTTACGACTTACTAAATCCCCACAAACAAATCAACTGAGAA
AA
SEQ ID NO:3 
ATTCTGGCCCTTGCACCTGATCGCGAAGGTGGAAATGGCAGAAGGATCAGCCTGGACGAAGCAACCAGTTCCAACTG
CTAAGTAAAGAAGATGCTAGACGAAGGAGACTTCAGAGGTGAAAAGTTTGCAAGAAGAGAGCTGCGGGAAATAAATT
TTCAATTTAAGGACTTGAGTGCGTCCATATTCGTGTACGTGTCCAACTGTTTTCCATTACCTAAGAAAAACATAAAG
ATTAAAAAGATAAACCCAATCGGGAAACTTTAGCGTGCCGTTTCGGATTCCGAAAAACTTTTGGAGCGCCAGATGAC
TATGGAAAGAGGAGTGTACCAAAATGGCAAGTCGGGGGCTACTCACCGGATAGCCAATACATTCTCTAGGAACCAGG
GATGAATCCAGGTTTTTGTTGTCACGGTAGGTCAAGCATTCACTTCTTAGGAATATCTCGTTGAAAGCTACTTGAAA
TCCCATTGGGTGCGGAACCAGCTTCTAATTAAATAGTTCGATGATGTTCTCTAAGTGGGACTCTACGGCTCAAACTT
CTACACAGCATCATCTTAGTAGTCCCTTCCCAAAACACCATTCTAGGTTTCGGAACGTAACGAAACAATGTTCCTCT
CTTCACATTGGGCCGTTACTCTAGCCTTCCGAAGAACCAATAAAAGGGACCGGCTGAAACGGGTGTGGAAACTCCTG
TCCAGTTTATGGCAAAGGCTACAGAAATCCCAATCTTGTCGGGATGTTGCTCCTCCCAAACGCCATATTGTACTGCA
GTTGGTGCGCATTTTAGGGAAAATTTACCCCAGATGTCCTGATTTTCGAGGGCTACCCCCAACTCCCTGTGCTTATA
CTTAGTCTAATTCTATTCAGTGTGCTGACCTACACGTAATGATGTCGTAACCCAGTTAAATGGCCGAAAAACTATTT
AAGTAAGTTTATTTCTCCTCCAGATGAGACTCTCCTTCTTTTCTCCGCTAGTTATCAAACTATAAACCTATTTTACC
TCAAATACCTCCAACATCACCCACTTAAACA
SEQ ID NO:4 
ATCTTCATTGATGAAACGTTGTGATCGGTGTGACTTTTATAGTAAAAGCTACAACTGTTTGAAATACCAAGATATCA
TTGTGAATGGCTCAAAAGGGTAATACATCTGAAAAACCTGAAGTGTGGAAAATTCCGATGGAGCCAACTCATGATAA
CGCAGAAGTCCCATTTTGCCATCTTCTCTTGGTATGAAACGGTAGAAAATGATCCGAGTATGCCAATTGATACTCTT
GATTCATGCCCTATAGTTTGCGTAGGGTTTAATTGATCTCCTGGTCTATCGATCTGGGACGCAATGTAGACCCCATT
AGTGGAAACACTGAAAGGGATCCAACACTCTAGGCGGACCCGCTCACAGTCATTTCAGGACAATCACCACAGGAATC
AACTACTTCTCCCAGTCTTCCTTGCGTGAAGCTTCAAGCCTACAACATAACACTTCTTACTTAATCTTTGATTCTCG
AATTGTTTACCCAATCTTGACAACTTAGCCTAAGCAATACTCTGGGGTTATATATAGCAATTGCTCTTCCTCGCTGT
AGCGTTCATTCCATCTTTCTA
SEQ ID NO:5 
TGTCATCTGCTGATGCTGTGAGGGAGAAAGAAGTAGGGGTGATACATGGTTTATAGGCAAAGCATGTTTGTTTCAGA
TCAAAGATTAGCGTTTCAAAGTTGTGGAAAAGTGACCATGCAACAATATGCAACACATTCGGATTATCTGATAAGTT
TCAAAGCTACTAAGTAAGCCCGTTTCAAGTCTCCAGACCGACATCTGCCATCCAGTGATTTTCTTAGTCCTGAAAAA
TACGATGTGTAAACATAAACCACAAAGATCGGCCTCCGAGGTTGAACCCTTACGAAAGAGACATCTGGTAGCGCCAA
TGCCAAAAAAAAATCACACCAGAAGGACAATTCCCTTCCCCCCCAGCCCATTAAAGCTTACCATTTCCTATTCCAAT
ACGTTCCATAGAGGGCATCGCTCGGCTCATTTTCGCGTGGGTCATACTAGAGCGGCTAGCTAGTCGGCTGTTTGAGC
TCTCTAATCGAGGGGTAAGGATGTCTAATATGTCATAATGGCTCACTATATAAAGAACCCGCTTGCTCAACCTTCGA
CTCCTTTCCCGATCCTTTGCTTGTTGCTTCTTCTTTTATAACAGGAAACAAAGGAATTTATACACTTTAA
SEQ ID NO:6 
AAATTAATCCATAAGATAAGGCAAATGTGCTTAAGTAATTGAAAACAGTGTTGTGATTATATAAGCATGGTATTTGA
ATAGAACTACTGGGGTTAACTTATCTAGTAGGATGGAAGTTGAGGGAGATCAAGATGCTTAAAGAAAAGGATTGGCC
AATATGAAAGCCATAATTAGCAATACTTATTTAATCAGATAATTGTGGGGCATTGTGACTTGACTTTTACCAGGACT
TCAAACCTCAACCATTTAAACAGTTATAGAAGACGTACCGTCACTTTTGCTTTTAATGTGATCTAAATGTGATCACA
TGAACTCAAACTAAAATGATATCTTTTACTGGACAAAAATGTTATCCTGCAAACAGAAAGCTTTCTTCTATTCTAAG
AAGAACATTTACATTGGTGGGAAACCTGAAAACAGAAAATAAATACTCCCCAGTGACCCTATGAGCAGGATTTTTGC
ATCCCTATTGTAGGCCTTTCAAACTCACACCTAATATTTCCCGCCACTCACACTATCAATGATCACTTCCCAGTTCT
CTTCTTCCCCTATTCGTACCATGCAACCCTTACACGCCTTTTCCATTTCGGTTCGGATGCGACTTCCAGTCTGTGGG
GTACGTAGCCTATTCTCTTAGCCGGTATTTAAACATACAAATTCACCCAAATTCTACCTTGATAAGGTAATTGATTA
ATTTCATAAAT
SEQ ID NO:7 
TCCAAACCAAACGGTCTAGCAAAAACGATAACTTTAAAGAACTTTTCAATTGGTTTTGTACACTACCACCGGTTTAC
TACCTCTGCCTTCGGTTCTTCTCCTCACATTTTTCGCAACTGGGATAGCGTAGCCTAAAGTGTCACATGCTCGCTGC
TCACATTCCCTACACAACAGAGATTGTCAGCAGAGGAAATTGAGCTCCACCATTCAACACTTGTGGATTTATGATAG
TCTGTGCTATCAGCTCTCTTTTTTTTGTTGCTGTAGAATTTACCGTGCTAGCAACCTTTTAAACTTTGTTTAGCTCT
CCTTCCCTCTTCCATTCATCTGTTTCGGTCCGATCCGTCTCTGGTCATCTCCTCCGCATTTTTTTTTTACCGTTAGC
GATAGGGGTCAGATCAATTCAATCAGTTTTGGCAAGGGTATTTAAAGGTGGCGAAATCCCCCTCCGTTTGTTGAACA
CATCCAACTATTCTCAACCCAACCATCTAACTAATCGTA
SEQ ID NO:8 
CGCAGCGTTTTCTGACGGTACTAGAGGACTCTTAGGGGAAGGTAGAATCAATAAAGATCATATTAGGTAAGCAAATT
TTGGATGGAATAGGAGACTAGGTGTGGATGCGCGATCTCGCCAAATTGCACGACCAGAGTGGATGCCGGATGGTGGT
AAACCGTTTCTTCCTTTTTACCACCCAAGTGCGAGTGAAACACCCCATGGCTGCTCTCCGATTGCCCCTCTACAGGC
ATAAGGGTGTGACTTTGTGGGCTTGAATTTTACACCCCCTCCAACTTTTCTCGCATCAATTGATCCTGTTACCAATA
TTGCATGCCCGGAGGAGACTTGCCCCCTAATTTCGCGGCGTCGTCCCGGATCGCAGGGTGAGACTGTAGAGACCCCA
CATAGTGACAATGATTATGTAAGAAGAGGGGGGTGATTCGGCCGGCTATCGAACTCTAACAACTAGGGGGGTGAACA
ATGCCCAGCAGTCCTCCCCACTCTTTGACAAATCAGTATCACCGATTAACACCCCAAATCTTATTCTCAACGGTCCC
TCATCCTTGCACCCCTCTTTGGACAAATGGCAGTTAGCATTGGTGCACTGACTGACTGCCCAACCTTAAACCCAAAT
TTCTTAGAAGGGGCCCATCTAGTTAGCGAGGGGTGAAAAATTCCTCCATCGGAGATGTATTGACCGTAAGTTGCTGC
TTAAAAAAAATCAGTTCAGATAGCGAGACTTTTTTGATTTCGCAACGGGAGTGCCTGTTCCATTCGATTGCAATTCT
CACCCCTTCTGCCCAGTCCTGCCAATTGCCCATGAATCTGCTAATTTCGTTGATTCCCACCCCCCTTTCCAACTCCA
CAAATTGTCCAATCTCGTTTTCCATTTGGGAGAATCTGCATGTCGACTACATAAAGCGACCGGTGTCCGAAAAGATC
TGTGTAGTTTTCAACATTTTGTGCTCCCCCCGCTGTTTGAAAACGGGGGTGAGCGCTCTCCGGGGTGCGAATTCGTG
CCCAATTCCTTTCACCCTGCCTATTGTAGACGTCAACCCGCATCTGGTGCGAATATAGCGCACCCCCAATGATCACA
CCAACAATTGGTCCACCCCTCCCCAATCTCTAATATTCACAATTCACCTCACTATAAATACCCCTGTCCTGCTCCCA
AATTCTTTTTTCCTTCTTCCATCAGCTACTAGCTTTTATCTTATTTACTTTACGAAA
SEQ ID NO:9 
TGTGTCTGTAAATTTAAATCCAAAGAAAAATAAAGGGCACTACATAGAACAACACAATCAACCTACATAGCTCTCTT
TTTTTTTTTTTTTTTTTTTTTCTTTTTTGTTTTTTCTCAATTTTCTTCATTTTTTGCATTGTTACTACTCTCCCATT
AAGGGAAATTCTACCAGGAAAACCGAAGAAGACAAATGCAAAAGGGAAATATGAATACGCATGTATGCGCGCAAAAC
CGCACTTACAGAGGGCATTAGGACATT
SEQ ID NO:10 
AGCAATGATATAAACAACAATTGAGTGACAGGTCTACTTTGTTCTCAAAAGGCCATAACCATCTGTTTGCATCTCTT
ATCACCACACCATCCTCCTCATCTGGCCTTCAATTGTGGGGAACAACTAGCATCCCAACACCAGACTAACTCCACCC
AGATGAAACCAGTTGTCGCTTACCAGTCAATGAATGTTGAGCTAACGTTCCTTGAAACTCGAATGATCCCAGCCTTG
CTGCGTATCATCCCTCCGCTATTCCGCCGCTTGCTCCAACCATGTTTCCGCCTTTTTCGAACAAGTTCAAATACCTA
TCTTTGGCAGGACTTTTCCTCCTGCCTTTTTTAGCCTCAGGTCTCGGTTAGCCTCTAGGCAAATTCTGGTCTTCATA
 
CCTATATCAACTTTTCATCAGATAGCCTTTGGGTTCAAAAAAGAACTAAAGCAGGATGCCTGATATATAAATCCCAG
ATGATCTGCTTTTGAAACTATTTTCAGTATCTTGATTCGTTTACTTACAAACAACTATTGTTGATTTTATCTGGAGA
ATAATCGAACAAA
SEQ ID NO:11 
CATAATGATATTTGAGGGTGTTAGTTACTTCGTCTCCAGAGTAGCTTATAGGGAGAAAAACCGAGACAACGATGGAA
CTCCCATGTAGATTCCACCGCCCCAATTACTGTTTTGGGCAATCCTGTTGATAAGACGCATTCTAGAGTTGTTTCAT
GAAAGGGTTACGGGTGTTGATTGGTTTGAGATATGCCAGAGGACAGATCAATCTGTGGTTTGCTAAACTGGAAGTCT
GGTAAGGACTCTAGCAAGTCCGTTACTCAAAAAGTCATACCAAGTAAGATTACGTAACACCTGGGCATGACTTTCTA
AGTTAGCAAGTCACCAAGAGGGTCCTATTTAACGTTTGGCGGTATCTGAAACACAAGACTTGCCTATCCCATAGTAC
ATCATATTACCTGTCAAGCTATGCTACCCCACAGAAATACCCCAAAAGTTGAAGTGAAAAAATGAAAATTACTGGTA
ACTTCACCCCATAACAAACTTAATAATTTCTGTAGCCAATGAAAGTAAACCCCATTCAATGTTCCGAGATTTAGTAT
ACTTGCCCCTATAAGAAACGAAGGATTTCAGCTTCCTTACCCCATGAACAGAAATCTTCCATTTACCCCCCACTGGA
GAGATCCGCCCAAACGAACAGATAATAGAAAAAAGAAATTCGGACAAATAGAACACTTTCTCAGCCAATTAAAGTCA
TTCCATGCACTCCCTTTAGCTGCCGTTCCATCCCTTTGTTGAGCAACACCATCGTTAGCCAGTACGAAAGAGGAAAC
TTAACCGATACCTTGGAGAAATCTAAGGCGCGAATGAGTTTAGCCTAGATATCCTTAGTGAAGGGTTGTTCCGATAC
TTCTCCACATTCAGTCATAGATGGGCAGCTTTGTTATCATGAAGAGACGGAAACGGGCATTAAGGGTTAACCGCCAA
ATTATATAAAGACAACATGTCCCCAGTTTAAAGTTTTTCTTTCCTATTCTTGTATCCTGAGTGACCGTTGTGTTTAA
TATAACAAGTTCGTTTTAACTTAAGACCAAAACCAGTTACAACAAATTATAACCCCTCTAAACACTAAAGTTCACTC
TTATCAAACTATCAAACATCAAAA
SEQ ID NO:12 
TGAGATAACAGAGTTGGGTAACTAGAGAGAATAATAGACGTATGCATGATTACTACACAACGGATGTCGCACTCTTT
CCTTAGTTAAAACTATCATCCAATCACAAGATGCGGGCTGGAAAGACTTGCTCCCGAAGGATAATCTTCTGCTTCTA
TCTCCCTTCCTCATATGGTTTCGCAGGGCTCATGCCCCTTCTTCCTTCGAACTGCCCGATGAGGAAGTCCTTAGCCT
ATCAAAGAATTCGGGACCATCATCGATTTTTAGAGCCTTACCTGATCGCAATCAGGATTTCACTACTCATATAAATA
CATCGCTCAAAGCTCCAACTTTGCTTGTTCATACAATTCTTGATATTCAC
SEQ ID NO:13 
ATTCTGGCCCTTGCACCTGATCGCGAAGGTGGAAATGGCAGAAGGATCAGCCTGGACGAAGCAACCAGTTCCAACTG
CTAAGTAAAGAAGATGCTAGACGAAGGAGACTTCAGAGGTGAAAAGTTTGCAAGAAGAGAGCTGCGGGAAATAAATT
TTCAATTTAAGGACTTGAGTGCGTCCATATTCGTGTACGTGTCCAACTGTTTTCCATTACCTAAGAAAAACATAAAG
ATTAAAAAGATAAACCCAATCGGGAAACTTTAGCGTGCCGTTTCGGATTCCGAAAAACTTTTGGAGCGCCAGATGAC
TATGGAAAGAGGAGTGTACCAAAATGGCAAGTCGGGGGCTACTCACCGGATAGCCAATACATTCTCTAGGAACCAGG
GATGAATCCAGGTTTTTGTTGTCACGGTAGGTCAAGCATTCACTTCTTAGGAATATCTCGTTGAAAGCTACTTGAAA
TCCCATTGGGTGCGGAACCAGCTTCTAATTAAATAGTTCGATGATGTTCTCTAAGTGGGACTCTACGGCTCAAACTT
CTACACAGCATCATCTTAGTAGTCCCTTCCCAAAACACCATTCTAGGTTTCGGAACGTAACGAAACAATGTTCCTCT
CTTCACATTGGGCCGTTACTCTAGCCTTCCGAAGAACCAATAAAAGGGACCGGCTGAAACGGGTGTGGAAACTCCTG
TCCAGTTTATGGCAAAGGCTACAGAAATCCCAATCTTGTCGGGATGTTGCTCCTCCCAAACGCCATATTGTACTGCA
GTTGGTGCGCATTTTAGGGAAAATTTACCCCAGATGTCCTGATTTTCGAGGGCTACCCCCAACTCCCTGTGCTTATA
CTTAGTCTAATTCTATTCAGTGTGCTGACCTACACGTAATGATGTCGTAACCCAGTTAAATGGCCGAAAAACTATTT
AAGTAAGTTTATTTCTCCTCCAGATGAGACTCTCCTTCTTTTCTCCGCTAGTTATCAAACTATAAACCTATTTTACC
TCAAATACCTCCAACATCACCCACTTAAACACGGCCGAATTCATAATGAGATTTCCTTCAATTTTTACTGCAGTTTT
ATTCGCAGCATCCTCCGCATTAGCTGCTCCAGTCAACACTACAACAGAAGATGAAACGGCACAAATTCCGGCTGAAG
CTGTCATCGGTTACTCAGATTTAGAAGGGGATTTCGATGTTGCTGTTTTGCCATTTTCCAACAGCACAAATAACGGG
TTATTGTTTATAAATACTACTATTGCCAGCATTGCTGCTAAAGAAGAAGGGGTATCTCTCGAGAAAAGAGAGGCTGA
AGCTTTAATTAACTGGGCCGCGAATTAATTCGCCTTAGACATGACTGTTCCTCAGTTCAAGTTGGGCACTTACGAGA
AGACCGGTCTTGCTAGATTCTAATCAAGAGGATGTCAGAATGCCATTTGCCTGAGAGATGCAGGCTTCATTTTTGAT
ACTTTTTTATTTGTAACCTATATAGTATAGGATTTTTTTTGTCATTTTGTTTCTTCTCGTACGAGCTTGCTCCTGAT
CAGCCTATCTCGCAGCTGATGAATATCTTGTGGTAGGGGTTTGGGAAAATCATTCGAGTTTGATGTTTTTCTTGGTA
TTTCCCACTCCTCTTCAGAGTACAGAAGATTAAGTGAGACGTTCGTTTGTGCAAGCTTATCGATAAGCTTTAATGCG
GTAGTTTATCACAGTTAAATTGCTAACGCAGTCAGGCACCGTGTATGAAATCTAACAATGCGCTCATCGTCATCCTC
GGCACCGTCACCCTGGATGCTGTAGGCATAGGCTTGGTTATGCCGGTACTGCCGGGCCTCTTGCGGGATATCGTCCA
TTCCGACAGCATCGCCAGTCACTATGGCGTGCTGCTAGGTACAACTTGAGCAAGTTGTCGATCAGCTCCTCAAATTG
 
GTCCTCTGTAACGGATGACTCAACTTGCACATTAACTTGAAGCTCAGTCGATTGAGTGAACTTGATCAGGTTGTGCA
GCTGGTCAGCAGCATAGGGAAACACGGCTTTTCCTACCAAACTCAAGGAATTATCAAACTCTGCAACACTTGCGTAT
GCAGGTAGCAAGGGAAATGTCATACTTGAAGTCGGACAGTGAGTGTAGTCTTGAGAAATTCTGAAGCCGTATTTTTA
TTATCAGTGAGTCAGTCATCAGGAGATCCTCTACGCCGGACGCATCGTGGCCGACCTGCAGGGGGGGGGGGGGCGCT
GAGGTCTGCCTCGTGAAGAAGGTGTTGCTGACTCATACCAGCTGAATCGCCCCATCATCCAGCCAGAAAGTGAGGGA
GCCACGGTTGATGAGAGCTTTGTTGTAGGTGGACCAGTTGGTGATTTTGAACTTTTGCTTTGCCACGGAACGGTCTG
CGTTGTCGGGAAGATGCGTGATCTGATCCTTCAACTCAGCAAAAGTTCGATTTATTCAACAAAGCCGCCGTCCCGTC
AAGTCAGCGTAATGCTCTGCCAGTGTTACAACCAATTAACCAATTCTGATTAGAAAAACTCATCGAGCATCAAATGA
AACTGCAATTTATTCATATCAGGATTATCAATACCATATTTTTGAAAAAGCCGTTTCTGTAATGAAGGAGAAAACTC
ACCGAGGCAGTTCCATAGGATGGCAAGATCCTGGTATCGGTCTGCGATTCCGACTCGTCCAACATCAATACAACCTA
TTAATTTCCCCTCGTCAAAAATAAGGTTATCAAGTGAGAAATCACCATGAGTGACGACTGAATCCGGTGAGAATGGC
AAAAGCTTATGCATTTCTTTCCAGACTTGTTCAACAGGCCAGCCATTACGCTCGTCATCAAAATCACTCGCATCAAC
CAAACCGTTATTCATTCGTGATTGCGCCTGAGCGAGACGAAATACGCGATCGCTGTTAAAAGGACAATTACAAACAG
GAATCGAATGCAACCGGCGCAGGAACACTGCCAGCGCATCAACAATATTTTCACCTGAATCAGGATATTCTTCTAAT
ACCTGGAATGCTGTTTTCCCGGGGATCGCAGTGGTGAGTAACCATGCATCATCAGGAGTACGGATAAAATGCTTGAT
GGTCGGAAGAGGCATAAATTCCGTCAGCCAGTTTAGTCTGACCATCTCATCTGTAACATCATTGGCAACGCTACCTT
TGCCATGTTTCAGAAACAACTCTGGCGCATCGGGCTTCCCATACAATCGATAGATTGTCGCACCTGATTGCCCGACA
TTATCGCGAGCCCATTTATACCCATATAAATCAGCATCCATGTTGGAATTTAATCGCGGCCTCGAGCAAGACGTTTC
CCGTTGAATATGGCTCATAACACCCCTTGTATTACTGTTTATGTAAGCAGACAGTTTTATTGTTCATGATGATATAT
TTTTATCTTGTGCAATGTAACATCAGAGATTTTGAGACACAACGTGGCTTTCCCCCCCCCCCCTGCAGGTCGGCATC
ACCGGCGCCACAGGTGCGGTTGCTGGCGCCTATATCGCCGACATCACCGATGGGGAAGATCGGGCTCGCCACTTCGG
GCTCATGAGCGCTTGTTTCGGCGTGGGTATGGTGGCAGGCCCCGTGGCCGGGGGACTGTTGGGCGCCATCTCCTTGC
ATGCACCATTCCTTGCGGCGGCGGTGCTCAACGGCCTCAACCTACTACTGGGCTGCTTCCTAATGCAGGAGTCGCAT
AAGGGAGAGCGTCGAGTATCTATGATTGGAAGTATGGGAATGGTGATACCCGCATTCTTCAGTGTCTTGAGGTCTCC
TATCAGATTATGCCCAACTAAAGCAACCGGAGGAGGAGATTTCATGGTAAATTTCTCTGACTTTTGGTCATCAGTAG
ACTCGAACTGTGAGACTATCTCGGTTATGACAGCAGAAATGTCCTTCTTGGAGACAGTAAATGAAGTCCCACCAATA
AAGAAATCCTTGTTATCAGGAACAAACTTCTTGTTTCGAACTTTTTCGGTGCCTTGAACTATAAAATGTAGAGTGGA
TATGTCGGGTAGGAATGGAGCGGGCAAATGCTTACCTTCTGGACCTTCAAGAGGTATGTAGGGTTTGTAGATACTGA
TGCCAACTTCAGTGACAACGTTGCTATTTCGTTCAAACCATTCCGAATCCAGAGAAATCAAAGTTGTTTGTCTACTA
TTGATCCAAGCCAGTGCGGTCTTGAAACTGACAATAGTGTGCTCGTGTTTTGAGGTCATCTTTGTATGAATAAATCT
AGTCTTTGATCTAAATAATCTTGACGAGCCAAGGCGATAAATACCCAAATCTAAAACTCTTTTAAAACGTTAAAAGG
ACAAGTATGTCTGCCTGTATTAAACCCCAAATCAGCTCGTAGTCTGATCCTCATCAACTTGAGGGGCACTATCTTGT
TTTAGAGAAATTTGCGGAGATGCGATATCGAGAAAAAGGTACGCTGATTTTAAACGTGAAATTTATCTCAAGATCTC
TGCCTCGCGCGTTTCGGTGATGACGGTGAAAACCTCTGACACATGCAGCTCCCGGAGACGGTCACAGCTTGTCTGTA
AGCGGATGCCGGGAGCAGACAAGCCCGTCAGGGCGCGTCAGCGGGTGTTGGCGGGTGTCGGGGCGCAGCCATGACCC
AGTCACGTAGCGATAGCGGAGTGTATACTGGCTTAACTATGCGGCATCAGAGCAGATTGTACTGAGAGTGCACCATA
TGCGGTGTGAAATACCGCACAGATGCGTAAGGAGAAAATACCGCATCAGGCGCTCTTCCGCTTCCTCGCTCACTGAC
TCGCTGCGCTCGGTCGTTCGGCTGCGGCGAGCGGTATCAGCTCACTCAAAGGCGGTAATACGGTTATCCACAGAATC
AGGGGATAACGCAGGAAAGAACATGTGAGCAAAAGGCCAGCAAAAGGCCAGGAACCGTAAAAAGGCCGCGTTGCTGG
CGTTTTTCCATAGGCTCCGCCCCCCTGACGAGCATCACAAAAATCGACGCTCAAGTCAGAGGTGGCGAAACCCGACA
GGACTATAAAGATACCAGGCGTTTCCCCCTGGAAGCTCCCTCGTGCGCTCTCCTGTTCCGACCCTGCCGCTTACCGG
ATACCTGTCCGCCTTTCTCCCTTCGGGAAGCGTGGCGCTTTCTCAATGCTCACGCTGTAGGTATCTCAGTTCGGTGT
AGGTCGTTCGCTCCAAGCTGGGCTGTGTGCACGAACCCCCCGTTCAGCCCGACCGCTGCGCCTTATCCGGTAACTAT
CGTCTTGAGTCCAACCCGGTAAGACACGACTTATCGCCACTGGCAGCAGCCACTGGTAACAGGATTAGCAGAGCGAG
GTATGTAGGCGGTGCTACAGAGTTCTTGAAGTGGTGGCCTAACTACGGCTACACTAGAAGGACAGTATTTGGTATCT
GCGCTCTGCTGAAGCCAGTTACCTTCGGAAAAAGAGTTGGTAGCTCTTGATCCGGCAAACAAACCACCGCTGGTAGC
GGTGGTTTTTTTGTTTGCAAGCAGCAGATTACGCGCAGAAAAAAAGGATCTCAAGAAGATCCTTTGATCTTTTCTAC
GGGGTCTGACGCTCAGTGGAACGAAAACTCACGTTAAGGGATTTTGGTCATGAGATTATCAAAAAGGATCTTCACCT
AGATCCTTTTAAATTAAAAATGAAGTTTTAAATCAATCTAAAGTATATATGAGTAAACTTGGTCTGACAGTTACCAA
TGCTTAATCAGTGAGGCACCTATCTCAGCGATCTGTCTATTTCGTTCATCCATAGTTGCCTGACTCCCCGTCGTGTA
GATAACTACGATACGGGAGGGCTTACCATCTGGCCCCAGTGCTGCAATGATACCGCGAGACCCACGCTCACCGGCTC
CAGATTTATCAGCAATAAACCAGCCAGCCGGAAGGGCCGAGCGCAGAAGTGGTCCTGCAACTTTATCCGCCTCCATC
CAGTCTATTAATTGTTGCCGGGAAGCTAGAGTAAGTAGTTCGCCAGTTAATAGTTTGCGCAACGTTGTTGCCATTGC
TGCAGGCATCGTGGTGTCACGCTCGTCGTTTGGTATGGCTTCATTCAGCTCCGGTTCCCAACGATCAAGGCGAGTTA
CATGATCCCCCATGTTGTGCAAAAAAGCGGTTAGCTCCTTCGGTCCTCCGATCGTTGTCAGAAGTAAGTTGGCCGCA
GTGTTATCACTCATGGTTATGGCAGCACTGCATAATTCTCTTACTGTCATGCCATCCGTAAGATGCTTTTCTGTGAC
TGGTGAGTACTCAACCAAGTCATTCTGAGAATAGTGTATGCGGCGACCGAGTTGCTCTTGCCCGGCGTCAACACGGG
 
ATAATACCGCGCCACATAGCAGAACTTTAAAAGTGCTCATCATTGGAAAACGTTCTTCGGGGCGAAAACTCTCAAGG
ATCTTACCGCTGTTGAGATCCAGTTCGATGTAACCCACTCGTGCACCCAACTGATCTTCAGCATCTTTTACTTTCAC
CAGCGTTTCTGGGTGAGCAAAAACAGGAAGGCAAAATGCCGCAAAAAAGGGAATAAGGGCGACACGGAAATGTTGAA
TACTCATACTCTTCCTTTTTCAATATTATTGAAGCATTTATCAGGGTTATTGTCTCATGAGCGGATACATATTTGAA
TGTATTTAGAAAAATAAACAAATAGGGGTTCCGCGCACATTTCCCCGAAAAGTGCCACCTGACGTC
SEQ ID NO:14 
AATAGTGACTCTGAATGCCCTTTGTCCCATGACGGATACTGTCTTCACGACGGCGTTTGTATGTATATCGAGGCACT
AGATAAGTATGCATGTAACTGCGTAGTAGGATACATTGGAGAACGTTGCCAATACCGTGATTTGAAGTGGTGGGAGT
TGCGT
SEQ ID NO:15 
TGGACGGGCGAGGACTCGGCGGAGCCCAACTCTGACTCGGCGGAGTGGATCCGAGACATGTACGCCAAGGTCACGGA
GATCTGGCAGGAGGTCATGCAGCGGCGGGACGACGACGGCGCGCTCCACGCCGCCTGCCAGGTGCAGCCGTCGGCCA
CGCTGGACGCCGCGCAGCCCCGGGTGACCGGCGTCGTCCTCTTCCGGCAGCTTGCGCCCCGCGCCAAGCTCGACGCC
TTCTTCGCCCTGGAGGGCTTCCCGACCGAGCCGAACAGCTCCAGCCGCGCCATCCACGTGCACCAGTTCGGGGACCT
GAGCCAGGGCTGCGAGTCCACCGGGCCCCACTACAACCCGCTGGCCGTGCCGCACCCGCAGCACCCGGGCGACTTCG
GCAACTTCGCGGTCCGCGACGGCAGCCTCTGGAGGTACCGCGCCGGCCTGGCCGCCTCGCTCGCGGGCCCGCACTCC
ATCGTGGGCCGGGCCGTGGTCGTCCACGCTGGCGAGGACGACCTGGGCCGCGGCGGTAACCAGGCCAGCGTGGAGAA
CGGGAACGCGGGCCGGCGGCTGGCCTGCTGCGTGGTGGGCGTGTGCGGGCCCGGGCTCTGGGAGCGCCAGGCGCGGG
AGCACTCAGAGCGCAAGAAGCGGCGGCGCGAGAGCGAGTGCAAGGCCGCC
SEQ ID NO:16 
GGCCGTAGGAGAAGGAGTGTTCAGTGGTGCGCCGTATCCCAACCCGAGGCCACAAAATGCTTCCAATGGCAAAGGAA
TATGAGAAAAGTGCGTGGCCCTCCTGTCAGCTGCATAAAGAGAGACTCCCCCATCCAGTGTATCCAGGCCATTGCGG
AAAACAGGGCCGATGCTGTGACCCTTGATGGTGGTTTCATATACGAGGCAGGCCTGGCCCCCTACAAACTGCGACCT
GTAGCGGCGGAAGTCTACGGGACCGAAAGACAGCCACGAACTCACTATTATGCCGTGGCTGTGGTGAAGAAGGGCGG
CAGCTTTCAGCTGAACGAACTGCAAGGTCTGAAGTCCTGCCACACAGGCCTTCGCAGGACCGCTGGATGGAATGTCC
CTATAGGGACACTTCGTCCATTCTTGAATTGGACGGGTCCACCTGAGCCCATTGAGGCAGCTGTGGCCAGGTTCTTC
TCAGCCAGCTGTGTTCCCGGTGCAGATAAAGGACAGTTCCCCAACCTGTGTCGCCTGTGTGCGGGGACAGGGGAAAA
CAAATGTGCCTTCTCCTCCCAGGAACCGTACTTCAGCTACTCTGGTGCCTTCAAGTGTCTGAGAGACGGGGCTGGAG
ACGTGGCTTTTATCAGAGAGAGCACAGTGTTTGAGGACCTGTCAGACGAGGCTGAAAGGGACGAGTATGAGTTACTC
TGCCCAGACAACACTCGGAAGCCAGTGGACAAGTTCAAAGACTGCCATCTGGCCCGGGTCCCTTCTCATGCCGTTGT
GGCACGAAGTGTGAATGGCAAGGAGGATGCCATCTGGAATCTTCTCCGCCAGGCACAGGAAAAGTTTGGAAAGGACA
AGTCACCGAAATTCCAGCTCTTTGGCTCCCCTAGTGGGCAGAAAGATCTGCTGTTCAAGGACTCTGCCATTGGGTTT
TCGAGGGTGCCCCCGAGGATAGATTCTGGGCTGTACCTTGGCTCCGGCTACTTCACTGCCATCCAGAACTTGAGGAA
AAGTGAGGAGGAAGTGGCTGCCCGGCGTGCGCGGGTCGTGTGGTGTGCGGTGGGCGAGCAGGAGCTGCGCAAGTGTA
ACCAGTGGAGTGGCTTGAGCGAAGGCAGCGTGACCTGCTCCTCGGCCTCCACCACAGAGGACTGCATCGCCCTGGTG
CTGAAAGGAGAAGCTGATGCCATGAGTTTGGATGGAGGATATGTGTACACTGCAGGCAAATGTGGTTTGGTGCCTGT
CCTGGCAGAGAACTACAAATCCCAACAAAGCAGTGACCCTGATCCTAACTGTGTGGATAGACCTGTGGAAGGATATC
TTGCTGTGGCGGTGGTTAGGAGATCAGACACTAGCCTTACCTGGAACTCTGTGAAAGGCAAGAAGTCCTGCCACACC
GCCGTGGACAGGACTGCAGGCTGGAATATCCCCATGGGCCTGCTCTTCAACCAGACGGGCTCCTGCAAATTTGATGA
ATATTTCAGTCAAAGCTGTGCCCCTGGGTCTGACCCGAGATCTAATCTCTGTGCTCTGTGTATTGGCGACGAGCAGG
GTGAGAATAAGTGCGTGCCCAACAGCAACGAGAGATACTACGGCTACACTGGGGCTTTCCGGTGCCTGGCTGAGAAT
GCTGGAGACGTTGCATTTGTGAAAGATGTCACTGTCTTGCAGAACACTGATGGAAATAACAATGAGGCATGGGCTAA
GGATTTGAAGCTGGCAGACTTTGCGCTGCTGTGCCTCGATGGCAAACGGAAGCCTGTGACTGAGGCTAGAAGCTGCC
ATCTTGCCATGGCCCCGAATCATGCCGTGGTGTCTCGGATGGATAAGGTGGAACGCCTGAAACAGGTGTTGCTCCAC
CAACAGGCTAAATTTGGGAGAAATGGATCTGACTGCCCGGACAAGTTTTGCTTATTCCAGTCTGAAACCAAAAACCT
TCTGTTCAATGACAACACTGAGTGTCTGGCCAGACTCCATGGCAAAACAACATATGAAAAATATTTGGGACCACAGT
ATGTCGCAGGCATTACTAATCTGAAAAAGTGCTCAACCTCCCCCCTCCTGGAAGCCTGTGAGTTCCTCAGGAAG
SEQ ID NO:20

GTACAGCCTACAGAAAGTATTGTCCGTTTCCCTAATATCACCAATTTATGCCCTTTTGGTGAAGTCTTCAATGCCAC
CAGATTCGCCTCCGTATATGCCTGGAACCGTAAGAGAATCAGTAATTGCGTGGCCGATTATTCTGTGTTGTATAATA
GTGCCTCCTTCTCCACCTTCAAGTGTTATGGCGTGTCCCCTACTAAGCTGAATGACTTATGTTTTACCAATGTTTAT
GCAGACTCATTCGTCATCCGTGGAGATGAAGTCCGTCAAATTGCCCCCGGACAGACAGGAAAAATCGCCGACTATAA
TTATAAACTGCCTGATGACTTCACCGGATGTGTCATCGCATGGAATAGTAATAATTTAGATTCAAAAGTAGGAGGAA
ATTATAATTATCTTTACCGTCTGTTTAGAAAATCAAACTTGAAACCATTCGAGAGAGATATTTCCACAGAGATCTAC
CAGGCAGGATCCACCCCTTGCAATGGAGTCGAAGGATTCAATTGCTATTTTCCTCTGCAGTCTTATGGATTTCAGCC
TACAAATGGAGTCGGATACCAACCTTATAGAGTCGTGGTCTTAAGTTTCGAGTTGCTTCACGCCGTCGCCACAGTGT
GCGGACCTAAAAAGTCAACAAATTTAGTCAAGAAT

### Amino Acid sequences

SEQ ID NO:17
   NSDSECPLSHDGYCLHDGVCMYIEALDKYACNCVVGYIGERCQYRDLKWWELR
SEQ ID NO:18 
WTGEDSAEPNSDSAEWIRDMYAKVTEIWQEVMQRRDDGALHAACQVQPSATLDAAQPRVTGVVLFRQLAPRAKLDA
FFALEGFPTEPNSSSRAIHVHQFGDLSQGCESTGPHYNPLAVPHPQHPGDFGNFAVRDGSLWRYRAGLAASLAGPHS
IVGRAVVVHAGEDDLGRGGNQASVENGNAGRRLACCVVGVCGPGLWERQAREHSERKKRRRESECKAA
SEQ ID NO:19 
GRRRRSVQWCAVSQPEATKCFQWQRNMRKVRGPPVSCIKRDSPIQCIQAIAENRADAVTLDGGFIYEAGLAPYKLRP
VAAEVYGTERQPRTHYYAVAVVKKGGSFQLNELQGLKSCHTGLRRTAGWNVPIGTLRPFLNWTGPPEPIEAAVARFF
SASCVPGADKGQFPNLCRLCAGTGENKCAFSSQEPYFSYSGAFKCLRDGAGDVAFIRESTVFEDLSDEAERDEYELL
CPDNTRKPVDKFKDCHLARVPSHAVVARSVNGKEDAIWNLLRQAQEKFGKDKSPKFQLFGSPSGQKDLLFKDSAIGF
SRVPPRIDSGLYLGSGYFTAIQNLRKSEEEVAARRARVVWCAVGEQELRKCNQWSGLSEGSVTCSSASTTEDCIALV
LKGEADAMSLDGGYVYTAGKCGLVPVLAENYKSQQSSDPDPNCVDRPVEGYLAVAVVRRSDTSLTWNSVKGKKSCHT
AVDRTAGWNIPMGLLFNQTGSCKFDEYFSQSCAPGSDPRSNLCALCIGDEQGENKCVPNSNERYYGYTGAFRCLAEN
AGDVAFVKDVTVLQNTDGNNNEAWAKDLKLADFALLCLDGKRKPVTEARSCHLAMAPNHAVVSRMDKVERLKQVLLH
QQAKFGRNGSDCPDKFCLFQSETKNLLFNDNTECLARLHGKTTYEKYLGPQYVAGITNLKKCSTSPLLEACEFLRK
SEQ ID NO:21

VQPTESIVRFPNITNLCPFGEVFNATRFASVYAWNRKRISNCVADYSVLYNSASFSTFKCYGVSPTKLNDLCFTNVY
ADSFVIRGDEVRQIAPGQTGKIADYNYKLPDDFTGCVIAWNSNNLDSKVGGNYNYLYRLFRKSNLKPFERDISTEIY
QAGSTPCNGVEGFNCYFPLQSYGFQPTNGVGYQPYRVVVLSFELLHAVATVCGPKKSTNLVKN

## Claims

1. A method for producing a transgenic cell product comprising:
(a) providing an expression system comprising: a methylotrophic yeast cell comprising an expression vector, said expression vector comprising an inducible MUT (Methanol Utilization) pathway promoter operably linked to a nucleic acid molecule encoding a recombinant protein of interest for producing a transgenic cell product of interest, wherein the inducible MUT promoter is selected from the group consisting of: NAD+-dependent formate dehydrogenase (FDH) promoter; alcohol oxidase 2 (AOX2) promoter; dihydroxyacetone kinase (DAK) promoter; dihydroxyacetone synthase 1,2 (DAS1,2) promoter; formaldehyde dehydrogenase 1 (FLD1) promoter; Fructose 1,6-bisphosphate aldolase (FBA) promoter; Peroxisomal membrane signal receptor PTS1 (PEX5) promoter; and catalase (CAT) promoter;
(b) growing the methylotrophic yeast cell on a suitable carbon source for supporting active growth of the methylotrophic yeast cell and for repressing the inducible MUT pathway promoter, thereby providing a methylotrophic yeast cell culture;
(c) after the methylotrophic yeast cell culture has attained a suitable methylotrophic yeast cell culture density, growing the methylotrophic yeast cell culture on a non-repressing carbon source selected from the group consisting of: sorbitol, mannitol, trehalose and alanine, thereby de-repressing the inducible MUT pathway promoter;
(d) adding an amount of an inducer compound selected from the group consisting of: S-formylglutathione; S-hydroxymethyl glutathione; formic acid; an alkali metal salt of formic acid; and an alkaline earth metal salt of formic acid; sufficient to induce the inducible MUT pathway promoter to the methylotrophic yeast cell culture such that the inducible MUT pathway promoter expresses the nucleic acid molecule encoding the recombinant protein of interest, thereby producing the transgenic cell product of interest; and
(e) recovering the transgenic cell product of interest from the methylotrophic yeast cell culture.

2. The method according to claim 1 wherein the methylotrophic yeast is selected from the group consisting of: *Pichia pastoris, Komagataella kurtzmanii, Komagataella phaffii, Pichia angusta, Pichia guillermordii, Pichia methanolica, Pichia inositovera, Hansenula polymorpha, Candida boidinii,* and *Yarrowia lipolytica.*

3. The method according to claim 1 wherein the methylotrophic yeast is *Pichia pastoris.*

4. The method according to claim 1 wherein steps (c), (d) and (e) are repeated more than once.

5. The method according to claim 1 wherein the non-repressing carbon source is initially added to the methylotrophic yeast cell culture in stages.

6. The method according to claim 4, wherein repeating steps (c), (d) and (e) more than once comprises, after the methylotrophic yeast cell culture has attained a suitable methylotrophic yeast cell culture density,
(i) adding to the methylotrophic yeast cell culture a non-repressing carbon source_selected from the group consisting of sorbitol, mannitol, trehalose and alanine,
(ii) adding an amount of an inducer compound selected from the group consisting of: S-formylglutathione; S-hydroxymethyl glutathione; formic acid; an alkali metal or ammonium salt of formic acid; and an alkaline earth metal salt of formic acid; sufficient to induce the inducible MUT pathway promoter to the methylotrophic yeast cell culture such that the inducible MUT pathway promoter initiates expression of the nucleic acid molecule encoding the recombinant protein of interest, thereby producing the transgenic cell product of interest; and
(iii) recovering the transgenic cell product of interest from the methylotrophic yeast cell culture; and
(iv) repeating steps (i) - (iii).

7. The method according to claim 6 wherein the non-repressing carbon source is initially added to the methylotrophic yeast cell culture in stages, starting prior to exhaustion of the growth repressing carbon source so that initially the methylotrophic yeast cell culture is growing on both the repressing carbon source and the non-repressing carbon source.

8. The method according to claim 6 wherein the inducer is added by doses or boluses in amount of 0.001-2.0g per 1 L of the culture up to 20 times a day.

9. The method according to claim 6 wherein the transgenic cell product of interest is recovered from the growth media and additional non-repressing carbon source and inducer compound are added to sustain growth of the methylotrophic yeast cell culture in batch phase so that the transgenic cell product of interest continues to be produced by the methylotrophic yeast cells and recovered from the media.

## Patentansprüche

1. Verfahren zur Herstellung eines transgenen Zellprodukts, umfassend:
(a) Bereitstellen eines Expressionssystems, umfassend: eine methylotrophe Hefezelle, die einen Expressionsvektor umfasst, wobei der Expressionsvektor einen induzierbaren MUT-(Methanol-Utilization)-Signalweg-Promotor umfasst, der funktionell mit einem Nukleinsäuremolekül verknüpft ist, das ein rekombinantes Protein von Interesse kodiert, um ein transgenes Zellprodukt von Interesse herzustellen, wobei der induzierbare MUT-Promotor aus der Gruppe ausgewählt ist, bestehend aus: NAD+-abhängiger Formiatdehydrogenase(FDH)-Promotor; Alkoholoxidase-2(AOX2)-Promotor; Peroxin-Pex14p(PEX14)-Promotor; Dihydroxyacetonkinase(DAK)-Promotor; Dihydroxyacetonsynthase-1,2(DAS1,2)-Promotor; Formylglutathiondehydrogenase(FGH)-Promotor; Formaldehyddehydrogenase-1(FLD1)-Promotor; Fruktose-1,6-bisphosphat-Aldolase(FBA)-Promotor; Peroxisomaler Membransignalrezeptor-PTS1(PEX5)-Promotor; Alkoholdehydrogenase-2(ADH2)-Promotor; und Katalase(CAT)-Promotor;
(b) Wachsen lassen der methylotrophen Hefezelle auf einer geeigneten Kohlenstoffquelle zur Unterstützung des aktiven Wachstums der methylotrophen Hefezelle und zur Unterdrückung des induzierbaren MUT-Signalweg-Promotors, wodurch eine methylotrophe Hefezellkultur bereitgestellt wird;
(c) nachdem die methylotrophe Hefezellkultur eine geeignete methylotrophe Hefezellkulturdichte erreicht hat, Wachsen lassen der methylotrophen Hefezellkultur auf einer nicht-reprimierenden Kohlenstoffquelle, ausgewählt aus der Gruppe, bestehend aus: Sorbitol, Mannitol, Trehalose und Alanin, wodurch der induzierbare MUT-Signalweg-Promotor dereprimiert wird;
(d) Hinzufügen einer Menge einer Induktorverbindung, ausgewählt aus der Gruppe, bestehend aus: S-Formylglutathion; S-Hydroxymethylglutathion; Ameisensäure; einem Alkalimetallsalz der Ameisensäure; und einem Erdalkalimetallsalz der Ameisensäure; ausreichend, um den induzierbaren MUT-Signalweg-Promotor zur methylotrophen Hefezellkultur zu induzieren, so dass der induzierbare MUT-Signalweg-Promotor das Nukleinsäuremolekül exprimiert, das das rekombinante Protein von Interesse kodiert, wodurch das transgene Zellprodukt von Interesse erzeugt wird; und
(e) Gewinnung des transgenen Zellprodukts von Interesse aus der methylotrophen Hefezellkultur.

2. Verfahren nach Anspruch 1, wobei die methylotrophe Hefe aus der Gruppe, bestehend aus *Pichia pastoris, Komagataella kurtzmanii, Komagataella phaffii, Pichia angusta, Pichia guillermordii, Pichia methanolica, Pichia inositovera, Hansenula polymorpha, Candida boidinii* und *Yarrowia lipolytica,* ausgewählt ist.

3. Verfahren gemäß Anspruch 1, wobei die methylotrophe Hefe *Pichia pastoris* ist.

4. Verfahren gemäß Anspruch 1, wobei die Schritte (c), (d) und (e) mehr als einmal wiederholt werden.

5. Verfahren nach Anspruch 1, wobei die nicht-reprimierende Kohlenstoffquelle zunächst stufenweise zur methylotrophen Hefezellkultur hinzugefügt wird.

6. Verfahren nach Anspruch 4, wobei das mehr als einmalige Wiederholen der Schritte (c), (d) und (e) umfasst,
nachdem die methylotrophe Hefezellkultur eine geeignete methylotrophe Hefezellkulturdichte erreicht hat:
(i) Hinzufügen einer nicht-reprimierenden Kohlenstoffquelle, ausgewählt aus der Gruppe, bestehend aus Sorbitol, Mannitol, Trehalose und Alanin, zu der methylotrophen Hefezellkultur,
(ii) Hinzufügen einer Menge einer Induktorverbindung, ausgewählt aus der Gruppe bestehend aus: S-Formylglutathion; S-Hydroxymethylglutathion; Ameisensäure; einem Alkalimetall- oder Ammoniumsalz von Ameisensäure; und einem Erdalkalimetallsalz von Ameisensäure; ausreichend, um den induzierbaren MUT-Signalweg-Promotor zur methylotrophen Hefezellkultur zu induzieren, so dass der induzierbare MUT-Signalweg-Promotor die Expression des Nukleinsäuremoleküls initiiert, das das rekombinante Protein von Interesse codiert, wodurch das transgene Zellprodukt von Interesse erzeugt wird; und
(iii) Gewinnen des transgenen Zellprodukts von Interesse aus der methylotrophen Hefezellkultur; und
(iv) Wiederholen der Schritte (i)-(iii).

7. Verfahren nach Anspruch 6, wobei die nicht-reprimierende Kohlenstoffquelle zunächst stufenweise zur methylotrophen Hefezellkultur hinzugefügt wird, beginnend vor der Erschöpfung der das Wachstum unterdrückenden Kohlenstoffquelle, so dass die methylotrophe Hefezellkultur zunächst sowohl auf der reprimierenden Kohlenstoffquelle als auch auf der nicht-reprimierenden Kohlenstoffquelle wächst.

8. Verfahren gemäß Anspruch 6, wobei der Induktor durch Dosen oder Boli in Mengen von 0,001 bis 2,0 g pro 1 L der Kultur bis zu 20-mal täglich hinzugefügt wird.

9. Verfahren nach Anspruch 6, wobei das transgene Zellprodukt von Interesse aus dem Wachstumsmedium gewonnen wird und zusätzliche nicht-reprimierende Kohlenstoffquellen und Induktorverbindungen hinzugefügt werden, um das Wachstum der methylotrophen Hefezellkultur in der Batch-Phase aufrechtzuerhalten, so dass das transgene Zellprodukt von Interesse weiterhin von den methylotrophen Hefezellen produziert und aus dem Medium gewonnen wird.

## Revendications

1. Procédé de production d'un produit cellulaire transgénique, comprenant :
(a) la préparation d'un système d'expression comprenant : une cellule de levure méthylotrophe comprenant un vecteur d'expression, ledit vecteur d'expression comprenant un promoteur inductible de voie MUT (utilisation de méthanol) fonctionnellement lié à une molécule d'acide nucléique codant une protéine recombinante d'intérêt pour la production d'un produit cellulaire transgénique d'intérêt, où le promoteur inductible MUT est sélectionné dans le groupe comprenant : promoteur de formate déshydrogénase (FDH) dépendante du NAD+ ; promoteur d'alcool oxydase 2 (AOX2) ; promoteur de dihydroxyacétone kinase (DAK) ; promoteur de dihydroxyacétone synthase 1,2 (DAS1,2) ; promoteur de formaldéhyde déshydrogénase 1 (FLD1) ; promoteur de fructose 1,6-bisphosphate aldolase (FBA) ; promoteur de récepteur du signal de membrane peroxysomale PTS1 (PEX5) ; et promoteur de catalase (CAT) ;
(b) culture cellulaire de levure méthylotrophe sur une source de carbone appropriée pour assister la croissance active de la cellule de levure méthylotrophe et pour la répression du promoteur inductible de voie MUT, permettant ainsi l'obtention d'une culture cellulaire de levure méthylotrophe ;
(c) après que la culture cellulaire de levure méthylotrophe a atteint une densité appropriée, culture de la culture cellulaire de levure méthylotrophe sur une source de carbone non répressive sélectionnée dans le groupe comprenant le sorbitol, le mannitol, le tréhalose et l'alanine, permettant ainsi la dérépression du promoteur inductible de voie MUT ;
(d) ajout d'une quantité d'un composé inducteur sélectionné dans le groupe comprenant : S-formylglutathione ; S-hydroxyméthylglutathione ; acide formique ; sel alcalin de l'acide formique ; et sel alcalino-terreux de l'acide formique ; suffisante pour induire le promoteur inductible de voie MUT à la culture cellulaire de levure méthylotrophe, de sorte que le promoteur inductible de voie MUT exprime la molécule d'acide nucléique codant la protéine recombinante d'intérêt, produisant ainsi le produit cellulaire transgénique d'intérêt ; et
(e) récupération du produit cellulaire transgénique d'intérêt à partir de la culture cellulaire de levure méthylotrophe.

2. Procédé selon la revendication 1, où la levure méthylotrophe est sélectionnée dans le groupe comprenant : *Pichia pastoris, Komagataella kurtzmanii, Komagataella phaffii, Pichia angusta, Pichia guillermordii, Pichia methanolica, Pichia inositovera, Hansenula polymorpha, Candida boidinii* et *Yarrowia lipolytica.*

3. Procédé selon la revendication 1, où la levure méthylotrophe est *Pichia pastoris.*

4. Procédé selon la revendication 1, où les étapes (c), (d) et (e) sont répétées plus d'une fois.

5. Procédé selon la revendication 1, où la source de carbone non répressive est initialement ajoutée par étapes à la culture cellulaire de levure méthylotrophe.

6. Procédé selon la revendication 4, où la répétition des étapes (c), (d) et (e) plus d'une fois comprend, après que la culture cellulaire de levure méthylotrophe a atteint une densité de culture cellulaire de levure méthylotrophe appropriée,
(i) l'ajout à la culture cellulaire de levure méthylotrophe d'une source de carbone non répressive sélectionnée dans le groupe comprenant le sorbitol, le mannitol, le tréhalose et l'alanine,
(ii) l'ajout d'une quantité d'un composé inducteur sélectionné dans le groupe comprenant : S-formylglutathione ; S-hydroxyméthylglutathione ; acide formique ; métal alcalin ou sel d'ammonium de l'acide formique ; et sel alcalino-terreux de l'acide formique ; suffisante pour induire le promoteur inductible de voie MUT à la culture cellulaire de levure méthylotrophe, de sorte que le promoteur inductible de voie MUT initie l'expression de la molécule d'acide nucléique codant la protéine recombinante d'intérêt, produisant ainsi le produit cellulaire transgénique d'intérêt ; et
(iii) la récupération du produit cellulaire transgénique d'intérêt à partir de la culture cellulaire de levure méthylotrophe ; et
(iv) la répétition des étapes (i) à (iii).

7. Procédé selon la revendication 6, où la source de carbone non répressive est initialement ajoutée par étapes à la culture cellulaire de levure méthylotrophe, en commençant avant l'épuisement de la source de carbone de croissance répressive, de sorte que la culture cellulaire de levure méthylotrophe se développe initialement à la fois sur la source de carbone répressive et sur la source de carbone non répressive.

8. Procédé selon la revendication 6, où l'inducteur est ajouté par doses ou bolus en quantité de 0,001 à 2,0 g par 1 L de la culture jusqu'à 20 fois par jour.

9. Procédé selon la revendication 6, où le produit cellulaire transgénique d'intérêt est récupéré à partir du milieu de croissance, et une source de carbone non répressive supplémentaire et un composé inducteur sont ajoutés pour assister la croissance de la culture cellulaire de levure méthylotrophe en phase discontinue, de sorte que le produit cellulaire transgénique d'intérêt continue à être produit par les cellules de levure méthylotrophe et à être récupéré à partir du milieu.
